# EUROPEAN PATENT APPLICATION

(11) **EP 4 477 666 A1**
(43) Date of publication of application: **18.12.2024**
(21) Application number: 23178906.6
(22) Date of filing: 13.06.2023
(51) Int. Cl.: C07K 16/18

(54) **ANTI-NPC VHH ANTIBODIES AND METHODS FOR THEIR STABILIZATION**

(71) Applicant: Max-Planck-Gesellschaft zur Förderung der Wissenschaften e.V., 80539 München (DE)
(72) Inventor: The designation of the inventor has not yet been filed
(74) Representative: Weickmann & Weickmann PartmbB

(57) **Abstract**

The present invention relates to VHH antibodies that specifically bind to components of nuclear pore complexes of human cells and other species. Further, the present invention relates to the labelling of VHH antibodies through maleimide chemistry and ectopic cysteine residues. Further, the present invention relates to a method for forming a stabilizing, structural disulfide bond in initially reduced VHH antibodies.

## Description

### Field of the Invention

The present invention relates to VHH antibodies that specifically bind to components of nuclear pore complexes of human cells and other species. Further, the present invention relates to the labelling of VHH antibodies through maleimide chemistry and ectopic cysteine residues. Further, the present invention relates to a method for forming a stabilizing, structural disulfide bond in initially reduced VHH antibodies.

### Background of the invention

VHH antibodies (VHHs, also called nanobodies) are isolated antigen-binding domains of camelid heavy-chain-only antibodies (Hamers-Casterman *et al.,* 1993; Muyldermans, 2013). They are straightforward to obtain by immunizing a camelid, such as an alpaca, with an antigen of choice, preparing an immune library, and performing phage display using the antigen again as a panning bait. Such workflow avoids the combinatorial complications typical of traditional antibodies whose paratopes are made of two different polypeptide chains.

VHH antibodies have found many practical applications, as crystallization chaperones, as imaging agents in immunofluorescence applications (Rothbauer *et al.,* 2008; Pleiner *et al.,* 2015, 2018), or for virus-neutralization in anti-viral therapy (Güttler *et al.,* 2021; Koenig *et al.,* 2021).

VHHs are easily produced in bacteria, such as *Escherichia coli,* or in yeast, such as *Pichia pastoris.* They contain a highly conserved structural disulfide bond that is buried in their hydrophobic core and stabilizes them against denaturation (Muyldermans, 2013; Pleiner *et al.,* 2015). This disulfide bond forms between two conserved cysteines, but only when the VHH is produced under the oxidizing conditions that prevail in a secretory compartment, such as the periplasm of *E. coli* or the endoplasmic reticulum of eukaryotes. Its formation is catalyzed by protein disulfide isomerases or PDIs for short (Goldberger et al., 1963; Roth and Mesirow, 1984; Edman et al., 1985; Lambert and Freedman, 1985; Bulleid and Freedman, 1988; Bardwell et al., 1991; Tu and Weissman, 2004; Hatahet and Ruddock, 2009).

Formation of the structural disulfide bond has, however, little direct effect on the VHH structure and its paratope. In fact, VHHs can recognize their antigens even when they are produced under reducing conditions in the cytoplasm, i.e., when their structural disulfide bond has not formed and the corresponding cysteines remained in their -SH form (Pleiner *et al.,* 2015).

The laboratory of the inventors exploited reducing expression previously to produce directly fluorophore-labeled, VHH-based imaging reagents (Pleiner *et al.,* 2015, 2018). For labeling, they coupled fluorophore-maleimides to engineered cysteines that were introduced, for example, at the N- and/or C-terminus of the respective VHH. The thereby occurring thiol Michael addition results in a stable thioether linkage between VHH and fluorophore(s), and it allows labeling without modifying the paratope. This requires, however, the acceptor cysteines to be reduced. This can be ensured by cytoplasmic production under reducing conditions. Indeed, such a workflow has yielded VHH imaging reagents of excellent performance (Pleiner *et al.,* 2015, 2018).

Fluorescent VHH imaging reagents are of particular interest because their actual production does not require animals and they are fully defined by sequence. Compared to traditional stainings with primary and secondary antibodies, they allow for simplified staining workflows (with just a single incubation of the specimen) and a smaller offset (linkage error) between label and epitopes. The latter is highly relevant for super-resolution microscopy, where the linkage error becomes limiting for resolution.

Nuclear pore complexes (NPCs) are a perfect and indeed preferred object for super-resolution microscopy (Löschberger *et al.,* 2012; Szymborska *et al.,* 2013; Pleiner *et al*., 2015; Göttfert *et al.,* 2017; Weber *et al.,* 2023). They are embedded into the nuclear envelope, conduct nucleocytoplasmic transport, have an outer diameter of 120 nm, and are of 8-fold rotational symmetry. When stained for immunofluorescence, NPCs yield diagnostic images, provided the resolution is sufficient to reveal such details. The laboratory of the inventors has previously reported VHHs recognizing Xenopus (i.e., frog) NPCs. While this was straightforward because Xenopus nucleoporins are very immunogenic in camelids, their use was limited because only a few labs work with frog cells, but many more with human! mammalian cell lines.

It was an object of the present invention to provide VHH antibodies showing improved binding to mammalian NPCs.

### Summary of the Invention

A first aspect of the present invention relates to an VHH antibody against a mammalian nuclear pore complex (NPC). In some embodiments, the VHH antibody is directed against a component of a mammalian NPC including Nup133, Nup358, Nup93, Nup35, and the Nup214·Nup88·Nup62 heterotrimer.

The VHH antibody comprises two structural cysteine residues capable of forming an intramolecular disulfide bond. In some embodiments, the VHH antibody comprises at least one additional solvent-accessible ectopic cysteine residue. A solvent-accessible ectopic cysteine residue may be introduced in any portion of the VHH antibody that does not interfere with target-recognition, for example, in its N- and/or C-terminal region and/or in the folded part of the VHH antibody avoiding the paratope regions. In particuar embodiments, the VHH antibody comprises a heterologous amino acid sequence including a solvent-accessible ectopic cysteine residue at its N-terminus and/or at its C-terminus.

In some embodiments, the VHH antibody carries at least one labelling agent, e.g., a fluorescent labelling agent (fluorophore).

In some embodiments, the VHH antibody carries a labelling agent attached to a solvent-accessible ectopic SH group on the VHH antibody, particularly an SH group on the N and/or C-terminus of the VHH antibody.

In some embodiments, the VHH antibody is a stable, e.g., a thermostabilized VHH antibody. In particular embodiments, the VHH antibody comprises two structural cysteines that are quantitatively oxidized to an intramolecular disulfide bond.

A further aspect of the present invention relates to a set of at least two VHH antibodies each recognizing a different component of a mammalian NPC selected from the group comprising Nup133, Nup358, Nup93, Nup35, and the Nup214·Nup88·Nup62 heterotrimer.

A further aspect of the present invention relates to the use of the VHH antibody or the set of the VHH antibodies for imaging applications including fluorescence spectroscopy or fluorescence microscopy.

The binding characteristics of specific VHH antibodies are shown in Table 1: All antibodies except Nb116 are directed against components of the NPC. Nb116 is directed against the Fc portion of rabbit IgG and may serve as a secondary detection reagent.

**Table 1**

| **VHH name** | **Target** | **Cross-reactivity** |
|---|---|---|
| KG1D08 | Nup133 β-propeller | Human-Xenopus |
| KG1C05 | Nup133 β-propeller | Human |
| KG2E04 | Nup107-Nup133 α-solenoid | Human-Xenopus |
| KG2B08 | Nup107_133 α-solenoid | Human-Xenopus |
| KG2B12 | Nup107_133 α-solenoid | Human-Xenopus |
| Re3E01 | Nup214·Nup88·Nup62 coiled coil trimer | Human-Xenopus |
| xhNup358-Nb2t | Nup358 | Human-Xenopus |
| xhNup93-Nb3t | Nup93 α-solenoid | Human-Xenopus |
| xhNup35-Nb1t | Nup35 RRM domain | Human-Xenopus |
| Nb116 | IgG Fc | Rabbit |

***Table 1**: VHH antibodies for imaging nuclear pores and their specificity*

The specificity-determining CDRs (complementarity-determining regions) of these VHH antibodies are shown in Table 2.

**Table 2**

| **VHH** | Seq ID | **CDR1** | Seq ID | **CDR2** | Seq ID | **CDR3** | Seq ID |
|---|---|---|---|---|---|---|---|
| KG1D08 | 1 | AASGRSFRGNIMA | 2 | ASITGSGYTSYENS | 3 | AGRFRGGHNRMPDAYEY | 4 |
| KG1C05 | 5 | ATSGILLSKAVMD | 6 | ARTGIRDSATYADF | 7 | KAAVTGVFGRLSEH | 8 |
| KG2E04 | 9 | AASGRGFSGYTWG | 10 | AAMSWITGSTYYADS | 11 | AARSKVSPDYDI | 12 |
| KG2B08 | 13 | AASGRGFSGYTWG | 14 | AAMSWITGSTYYADS | 15 | AARSKVSYDYDI | 16 |
| KG2B12 | 17 | AASGRGFSGYTWG | 18 | AAMSWITGSTYYADS | 19 | AARSKVSYDYDI | 20 |
| Re3E01 | 21 | AASGFTFTDYLMA | 22 | AYITSDGSTVYASS | 23 | SAKSGLKDY | 24 |
| xhNup358-Nb2t | 25 | AASGRPLSDYAMG | 26 | AAISW KGGETFYAGS | 27 | AAVLGSPHYSGTHYYPAEYDY | 28 |
| xhNup93-Nb3t | 29 | LFSGRPGRVFGHYLMG | 30 | AAISFSGDPTYYADS | 31 | AADRLPYGDSWASKDEYDY | 32 |
| xhNup35-Nb1t | 33 | VGTGNISSAYTMA | 34 | AGILNGGSTHYADS | 35 | HVVIESTRFVYDHY | 36 |
| Nb116 | 37 | TVSRIINWTRMG | 38 | ANIDPSGTTNYADF | 39 | FTIEPDRGTN | 40 |

***Table 2**: Specific VHHs and their CDR regions.*

A list of complete sequences of VHH antibodies (without ectopic cysteines) is shown at the end of the specification.

There are several methods known in the art for determining the CDR sequences of a given antibody molecule, but there is no standard unequivocal method. Determination of CDR sequences from antibody heavy chain variable regions can be made according to any method known in the art, including, but not limited to, the methods known as KABAT, Chothia, and IMGT. A selected set of CDRs may include sequences identified by more than one method, namely, some CDR sequences may be determined using KABAT and some using IMGT, for example. According to some embodiments of the present invention, the CDR sequences of the VHH variable regions are determined using the KABAT methods. CDRs may also be defined through a multiple alignment (with many other VHH antibodies), to identify the hot-spots of variability and relate them to a standard VHH antibody structure. It is also possible to define CDRs by analyzing the structure of the VHH antibody and deciding what is a loop and what is the antibody's scaffold. In some cases, CDR-adjacent residues are also variable, and are therefore included in the CDR definition.

A further aspect of the present invention relates to a method of producing an VHH antibody comprising the steps:
(i) expressing a VHH antibody comprising two structural cysteines and optionally at least one solvent-accessible ectopic cysteine in a host cell under reducing or at least partially reducing conditions,
(ii) purifying the VHH antibody from the host cell or the culture medium under conditions wherein the at least one solvent-accessible ectopic cysteine, if present, is in the reduced SH state;
(iii) optionally attaching a labelling agent to the at least one solvent-accessible ectopic cysteine under conditions wherein the structural cysteines are kept unmodified,
(iv)subjecting the VHH antibody to an oxidation that converts the two structural cysteines to a disulfide bond, and
(v) obtaining a thermostabilized VHH antibody wherein the two structural cysteines are quantitatively oxidized to a disulfide bond.

In some embodiments, the VHH antibody comprises two structural cysteines and at least one solvent-accessible ectopic cysteine. In some of those embodiments, the method comprises step (iii).

The oxidation in step (iv) may be carried out in the presence of an oxidation agent, e.g., a disulfide compound such as glutathione disulfide (GSSG) and optionally in the presence of a protein disulfide isomerase (PDI).

In some embodiments, the oxidation comprises a treatment at an elevated temperature thereby leading to a transient at least partial thermal unfolding of the VHH antibody. In particular embodiments, the VHH antibody is subjected to a treatment at a temperature which is at least as high as the onset of melting of the VHH antibody with reduced structural cysteines.

### Embodiments of the invention

In the following, specific embodiments of the invention are disclosed as follows:
1. A VHH antibody recognizing a component of a mammalian nuclear pore complex (NPC) selected from the group comprising Nup133, Nup358, Nup93, Nup35, and the Nup214·Nup88·Nup62 heterotrimer comprising:
   (a) a CDR3 sequence as shown in SEQ. ID NO: 2, 6, 10, 14, 18, 22, 26, 30, or 34;
   (b) a CDR3 sequence, which has an identity of at least 80%, at least 90% or at least 95% to a CDR3 sequence of (a); or
   (c) a VHH antibody, which competes with a VHH antibody of (a) for the binding to the respective component of the NPC.
2. The VHH antibody of embodiment 1 comprising
   (a) a combination of CDR1, CDR2 and CDR3 sequences as shown in SEQ. ID NO: 2-4, 6-8, 10-12, 14-16, 18-20, 22-24, 26-28, 30-32, or 34-36;
   (b) a combination of CDR1, CDR2 and CDR3 sequences which has an identity of at least 80%, at least 90% or at least 95% to a combination of CDR1, CDR2 and CDR3 sequences of (a); or
   (c) a VHH antibody, which competes with a VHH antibody of (a) for the binding to the respective component of the NPC.
3. The VHH antibody of embodiment 1 or 2 comprising
   (a) a VHH sequence as shown in SEQ. ID NO: 1, 5, 9, 13, 17, 21, 25, 29, or 33, and optionally at least one additional solvent-accessible ectopic cysteine residue;
   (b) a VHH sequence which has an identity of at least 70%, at least 80%, at least 90%, at least 95% or at least 98% to a VHH sequence of (a), or
   (c) a VHH antibody, which competes with a VHH antibody of (a) for the binding to the respective component of the NPC.
4. The VHH antibody of any one of embodiments 1-3 comprising at least one solvent-accessible ectopic cysteine residue in its N-and/or C-terminal region, particularly at its N- and/or C-terminus and/or in its folded part avoiding the paratope regions.
5. The VHH antibody of any one of embodiments 1-4 comprising a heterologous amino acid sequence including a solvent-accessible ectopic cysteine residue at its N-terminus and/or at its C-terminus.
6. The VHH antibody of any one of embodiments 1-5 which carries a labelling agent, particularly a fluorescent group, biotin, a DNA oligonucleotide group, an enzyme, an electron spin resonance (EPR) probe, or a group for immobilization to a chromatographic support.
7. The VHH antibody of any one of embodiments 1-6 which carries a labelling agent attached to a solvent-accessible ectopic SH group on the VHH antibody.
8. The VHH antibody of embodiment 7 wherein the labelling agent is attached as a thiol-reactive compound, e.g., an maleimide-, iodoacetamide-, bromoacetamide- or acrylamide-activated compound to a solvent-accessible ectopic SH group on the VHH antibody.
9. The VHH antibody of any one of embodiments 1-8 which is a thermostabilized VHH antibody.
10. The VHH antibody of any one of embodiments 1-9 which comprises two structural cysteines that are quantitatively oxidized and form an intramolecular disulfide bond.
11. The VHH antibody of any one of embodiments 1-10 which has a cross-reactivity with an NPC component from Xenopus laevis.
12. A set of at least 2 VHH antibodies recognizing each a different component of a mammalian nuclear pore complex (NPC) selected from the group comprising Nup133, Nup358, Nup93, Nup35, and the Nup214·Nup88·Nup62 heterotrimer.
13. The set of embodiment 12 wherein at least one VHH antibody comprises at least one solvent-accessible ectopic cysteine residue.
14. The set of embodiment 12 or 13 wherein at least one VHH antibody comprises a solvent-accessible ectopic cysteine residue in its N-and/or C-terminal region and/or in its folded part avoiding the paratope regions.
15. The set of any one of embodiments 12-14 comprising at least one VHH antibody comprising:
   (a) a CDR3 sequence as shown in SEQ. ID NO: 2, 6, 10, 14, 18, 22, 26, 30, or 34;
   (b) a CDR3 sequence, which has an identity of at least 80%, at least 90% or at least 95% to a CDR3 sequence of (a); or
   (c) a VHH antibody, which competes with a VHH antibody of (a) for the binding to the respective component of the NPC.
16. The set of any one of embodiments 12-15 comprising at least one VHH antibody comprising:
   (a) a combination of CDR1, CDR2 and CDR3 sequences as shown in SEQ. ID NO: 2-4, 6-8, 10-12, 14-16, 18-20, 22-24, 26-28, 30-32, or 34-36;
   (b) a combination of CDR1, CDR2 and CDR3 sequences which has an identity of at least 80%, at least 90% or at least 95% to a combination of CDR1, CDR2 and CDR3 sequences of (a); or
   (c) a VHH antibody, which competes with a VHH antibody of (a) for the binding to the respective component of the NPC.
17. The set of any one of embodiments 12-16 comprising at least one VHH antibody comprising:
   (a) a VHH sequence as shown in SEQ. ID NO: 1, 5, 9, 13, 17, 21, 25, 29, or 33; and optionally at least one additional solvent-accessible ectopic cysteine residue;
   (b) a VHH sequence which has an identity of at least 70%, at least 80%, at least 90%, at least 95% or at least 98% to a VHH sequence of (a), or
   (c) a VHH antibody, which competes with a VHH antibody of (a) for the binding to the respective component of the NPC.
18. The set of any one of embodiments 12-17 comprising at least one VHH antibody comprising 2, 3, 4 or 5 different VHH antibodies, particularly 2, 3, 4 or 5 different VHH antibodies as defined in any one of embodiments 13-15.
19. The set of any one of embodiments 12-18 wherein at least one VHH antibody carries a labelling agent, particularly a fluorescent group, biotin, a DNA oligonucleotide group, an enzyme, an electron spin resonance (EPR) probe, or a group for immobilization to a chromatographic support.
20. The set of embodiment 19 wherein at least 2 VHH antibodies, e.g., 2, 3, 4, or 5 VHH antibodies carry different labelling agents, particularly different spectrally separatable fluorescent groups.
21. The set of any one of embodiments 12-20 wherein at least one VHH antibody carries a labelling agent attached to a solvent-accesible ectopic SH group on the VHH antibody.
22. The set of any one of embodiments 12-21 wherein at least one VHH antibody is a thermostable VHH antibody.
23. The set of any one of embodiments 12-22 wherein at least one VHH antibody comprises two structural cysteines that are quantitatively oxidized and form an intramolecular disulfide bond.
24. Use of the VHH antibody of any one of embodiments 1-11 or the set of any one of embodiments 12-23 for imaging applications particularly for fluorescence spectroscopy or microscopy.
25. A method of producing a VHH antibody comprising the steps:
   (i) expressing a VHH antibody comprising two structural cysteines and optionally at least one solvent-accessible ectopic cysteine in a host cell under reducing or at least partially reducing conditions,
   (ii) purifying the VHH antibody from the host cell or the culture medium under conditions wherein the at least one solvent-accessible ectopic cysteine, if present, is in the reduced SH state;
   (iii) optionally attaching a labelling agent to the at least one solvent-accessible ectopic cysteine under conditions wherein the two structural cysteines are kept unmodified,
   (iv)subjecting the labelled VHH antibody to an oxidation that converts the two structural cysteines to a disulfide bond, and
   (v) obtaining a thermostabilized VHH antibody wherein the two structural cysteines are quantitatively oxidized to a disulfide bond.
26. The method of embodiment 25 wherein the VHH antibody comprises at least one solvent-accessible ectopic cysteine.
27. The method of embodiment 25 or 26 wherein the VHH antibody comprises an solvent-accessible ectopic cysteine residue in its N-and/or C-terminal region and/or in its folded part, avoiding the paratope regions.
28. The method of any one of embodiments 25 -27 wherein the VHH antibody comprises at least one heterologous solvent-accessible ectopic cysteine-containing amino acid sequence at its N- and/or C-terminus, particularly having a length of up to about 20 amino acids.
29. The method of any one of embodiments 25 -28 wherein the VHH antibody further comprises at least one heterologous tag-containing heterologous amino acid sequence at its N- and/or C-terminus, particularly a cleavable tag-containing amino acid sequence.
30. The method of any one of embodiments 25 -29 wherein the reducing or at least partially reducing conditions in step (i) comprise expression of the VHH antibody in the cytoplasm of a prokaryotic host cell such as *E.coli* or a eukaryotic host cell such as a yeast, protozoan, or animal cell, optionally followed by a reduction treatment.
31. The method of any one of embodiments 25-30 wherein step (ii) comprises a treatment with a reducing agent such as a thiol, e.g., DTT (dithiothreitol) or a phosphine, e.g., TCEP (tris(carboxyethyl)phosphine), followed by a removal of the reducing agent.
32. The method of any one of embodiments 25-30 wherein step (ii) comprises a chromatographic purification in the presence of a reducing agent.
33. The method of any one of embodiments 25-32 wherein the labelling agent is a thiol-reactive compound, e.g., an maleimide-, iodoacetamide-, bromoacetamide- or acrylamide-activated compound.
34. The method of any one of embodiments 25-33 wherein the labelling agent is a fluorophore, biotin, a DNA oligonucleotide, an enzyme, an electron spin resonance (EPR) probe, or a compound for immobilization to a chromatographic support.
35. The method of any one of embodiments 25-34 wherein step (iv) comprises a treatment of the VHH antibody with an oxidant.
36. The method of embodiment 35 wherein the oxidant is a disulfide compound particularly oxidized glutathione (GSSG).
37. The method of any one of embodiments 25-36 wherein the oxidation in step (iv) is carried out in the presence of a protein disulfide isomerase (PDI), particularly in the presence of DsbA and/or DsbC from *E. coli.*
38. The method of any one of embodiments 25-37 wherein step (iv) comprises a transient thermal unfolding of the VHH antibody.
39. The method of embodiment 38 wherein the transient thermal unfolding comprises incubating the VHH antibody at a temperature which is in the range between the onset of melting of the reduced form of the VHH antibody (with reduced structural cysteines) and the nominal Tm of the reduced form of the VHH antibody.
40. A VHH antibody recognizing an Fc region of a rabbit IgG comprising:
   (a) a CDR3 sequence as shown in SEQ. ID NO: 38;
   (b) a CDR3 sequence, which has an identity of at least 80%, at least 90% or at least 95% to the CDR3 sequence of (a); or
   (c) a VHH antibody, which competes with the VHH antibody of (a) for the binding to its target antigen.
41. The VHH antibody of embodiment 40 comprising
   (a) a combination of CDR1, CDR2 and CDR3 sequences as shown in SEQ. ID NO: 38-40;
   (b) a combination of CDR1, CDR2 and CDR3 sequences which has an identity of at least 80%, at least 90% or at least 95% to the combination of CDR1, CDR2 and CDR3 sequences of (a); or
   (c) a VHH antibody, which competes with a VHH antibody of (a) for the binding to its target antigen.
42. The VHH antibody of embodiment 40 or 41 comprising
   (a) a VHH sequence as shown in SEQ. ID NO: 37; and optionally an additional cysteine residue at its N- and/or C-terminus;
   (b) a sequence which has an identity of at least 70%, at least 80%, at least 90%, at least 95% or at least 99% to a VHH sequence of (a), or
   (c) a VHH antibody, which competes with a VHH antibody of (a) for the binding to its target antigen.
43. Use of the VHH antibody of any one of embodiments 40-42 as a secondary detection reagent, particularly for the imaging of an NPC, more particularly for a use as defined in embodiment 24.

### Detailed description of the invention

The inventors prepared mixes of human and Xenopus NPC antigens (Nup133, Nup358, Nup93, Nup35, the Nup214·Nup88·Nup62 heterotrimer), immunized alpacas, selected VHHs by phage display, produced them recombinantly, and labeled them fluorescent dyes. The VHH sequences of the antibodies are shown at the end of the description. Table 2 discloses their specificity-determining CDRs.

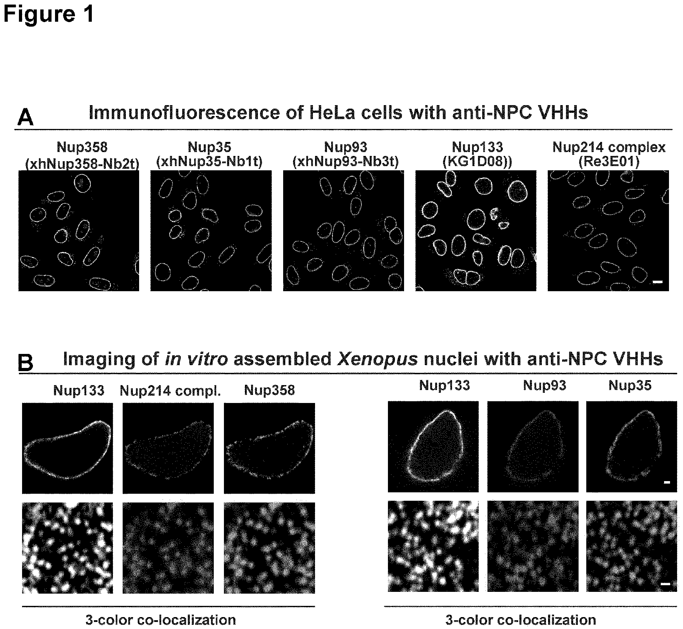

As detailed in Table 1 and Figure 1, several of the VHH antibodies actually performed very well in immunofluorescence applications and specifically recognize not only human NPCs, but also cross-react from frogs to humans. Images obtained by confocal laser scanning microscopy on human HeLa cells with the focal plane across the equator of the cell nuclei are shown in Figure 1A. The bright staining of the nuclear rim is indeed indicative of a specific detection of NPCs. In Figure 1B and C, stains of Xenopus nuclei are shown, demonstrating the cross-reaction over a long evolutionary distance. For staining, three VHHs (labelled with different fluorophores) were each combined, demonstrating their utility in multi-color co-localizations.

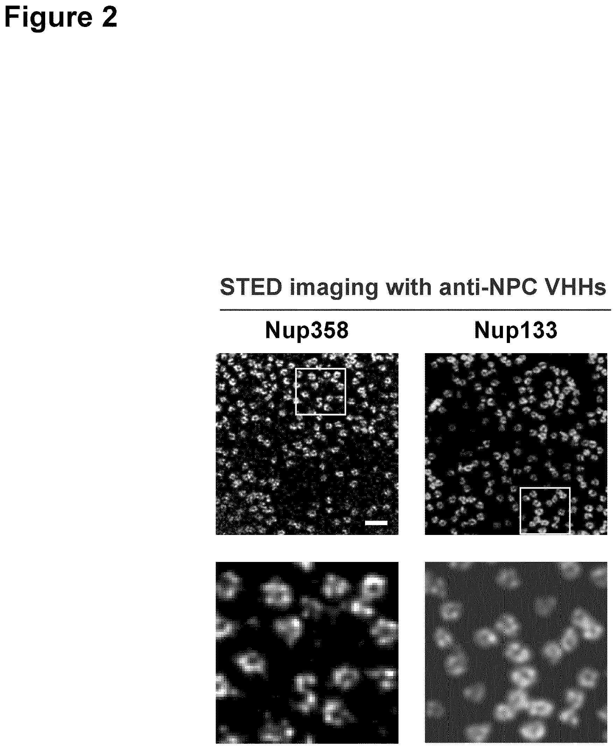

To obtain images at higher resolution, the inventors turned to STED (stimulated emission depletion) microscopy (Sahl et al., 2017). As documented for the anti-Nup358 and anti-Nup133 VHHs, this now allowed to resolve rings in individual NPCs (Figure 2), consistent with their 8-fold rotational symmetry. Individual Nups occur within NPCs at different positions and copy numbers (Hampoelz et al., 2019). The here-disclosed anti-Nup VHHs can thus be combined into sets, where individual VHHs are conjugated to compatible fluorophores of different spectral (excitation and/or emission) properties and used to generate 2D and 3D multicolor nanoscopic patterns. For example, one can combine Alexa488-labelled anti-Nup133 with AbberiorStar635-labelled anti-Nup358, and Alexa568-labelled anti-Nup35 to generate a three-color three-dimensional pattern by staining NPCs of HeLa cells. As such patterns can be reproduced by standardized cell-staining protocols, they can also be used for benchmarking super-resolution fluorescence microscopes. Here, it is relevant that such patterns are generated with biologically relevant samples, which can reveal typical challenges, such as offsets between color channels due to mismatches in diffractive index within the specimen.

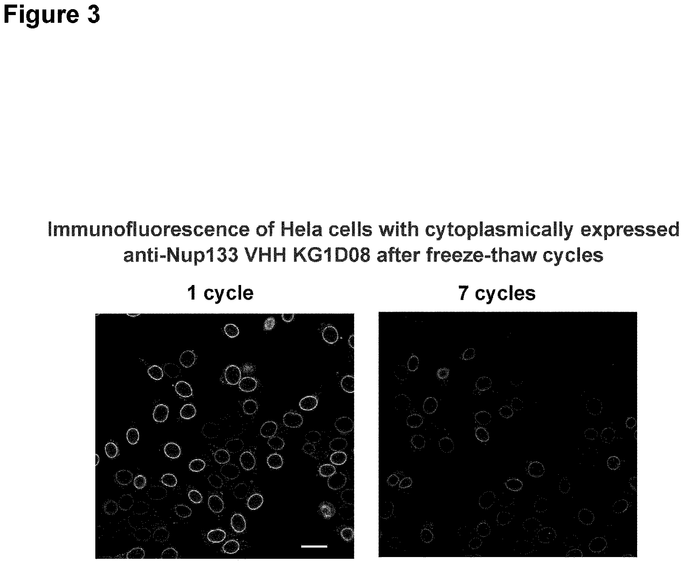

The inventors noted, however, that several of the VHHs started to denature and precipitate during repeated freeze-thaw cycles, during storage, or during prolonged incubations at room temperature or at 37°C. Figure 3 documents such problem for the anti-Nup133 VHH KG1D08 (SEQ ID NO: 1) labelled with AbberiorStar 635, which loses most of its staining-activity after 7 freeze-thaw cycles. This happens even in a buffer containing 250 mM sucrose, which normally confers already some protection against such damage.

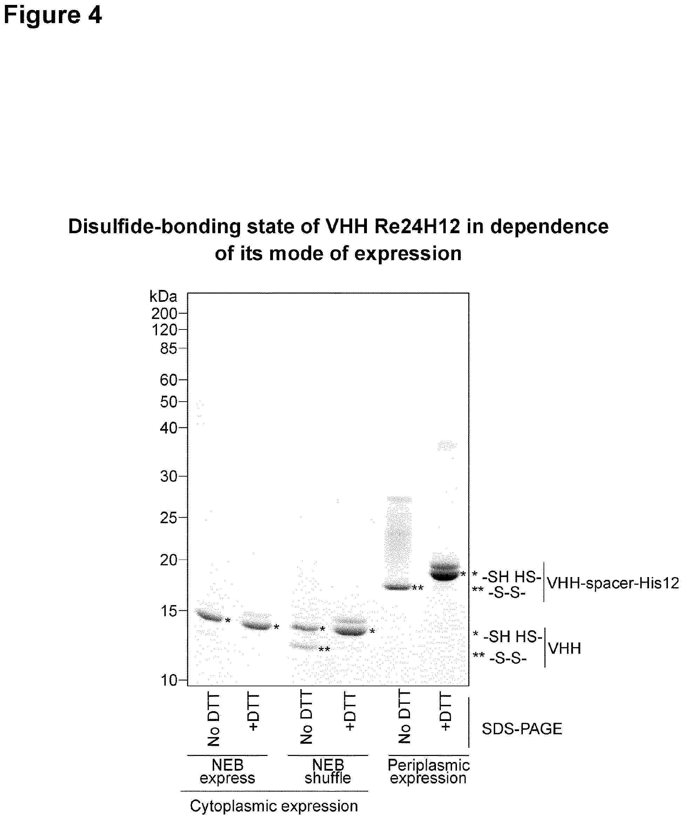

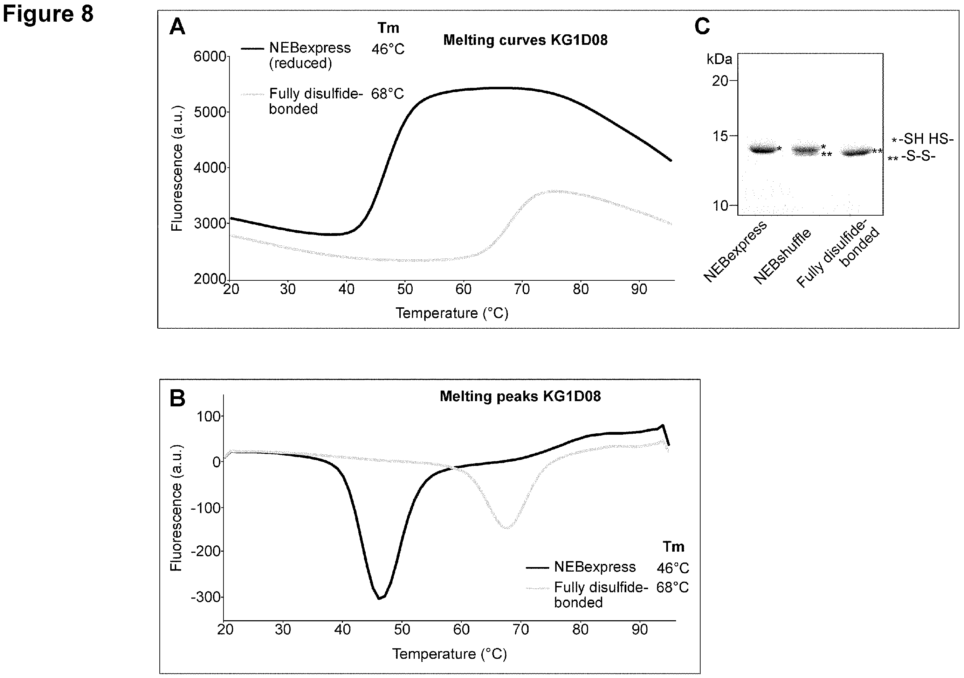

They reasoned that this reduced stability might be due to the structural disulfide bond not being formed and that the problem can be solved by oxidizing the two structural cysteines to cystine. One way of achieving this is to use the E. *coli* Shuffle strain (commercially available from NEB) whose cytoplasm is less reducing for disulfide bonds (because of a genetic deletion of the GOR gene, which encodes the glutathione reductase or more correctly a glutathione disulfide reductase). Such strain also expresses the PDI DsbC in the cytoplasm (Bessette *et al.,* 1999), which in wildtype cells forms disulfide bonds in the periplasm. However, the disulfide bond formation of VHHs expressed in Shuffle remains notoriously incomplete (see Figure 4 for VHH Re24H12 and below Figure 8C for VHH KG1D08). Furthermore, the Shuffle strain suffers from sensitivity already to atmospheric levels of oxygen and has a severely problematic growth phenotype or even completely fails to grow, for example, when inoculated at too low cell densities.

The inventors developed, therefore, an alternative technology. They developed a workflow that includes (i) expression of a VHH with ectopic cysteines for later labeling, under reducing (or semi-reducing) conditions for expression, (ii) VHH purification including a final reduction step to ensure an -SH state of the ectopic cysteines, (iii) modification of the ectopic (and fully exposed) cysteines with a label, e.g., a maleimide-activated label (fluorophore, biotin, DNA-oligonucleotide) while keeping the structural cysteines unmodified, (iv) a post-treatment that converts the two structural cysteines (which are buried in the hydrophobic core of the VHH) to a disulfide bond, and (v) obtaining the VHH antibody in a stable oxidized form.

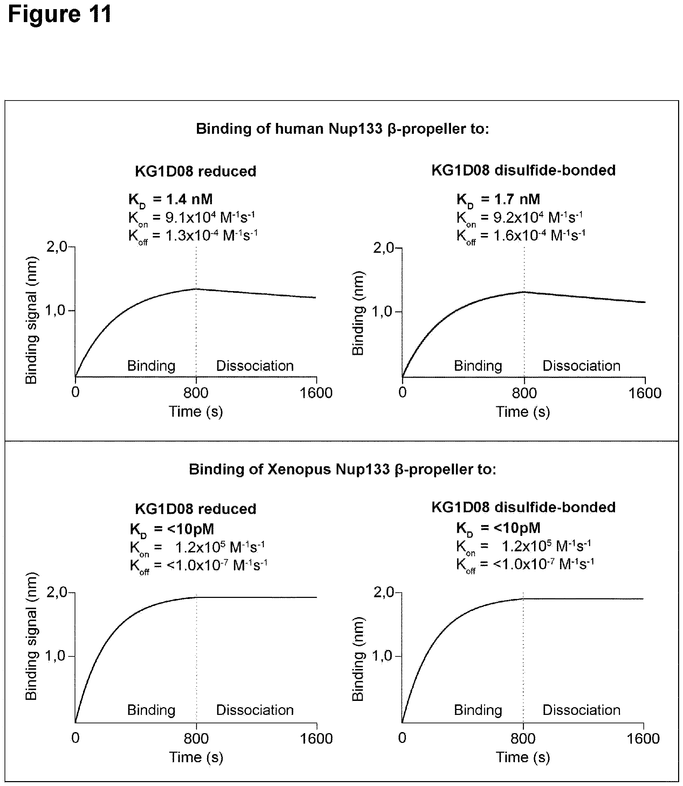

A PDI should catalyze the disulfide bond formation; however, initial experiments showed only low efficiency and rather variable results with VHHs. The inventors reasoned that the problem might relate to the fact that the two structural cysteines are buried in the hydrophobic interior of the VHH and are thus not easily accessible for modification or enzymatic action. This burial helps to confine the fluorophore-labelling to only the engineered, ectopic cysteines. However, in the next step of the above-sketched workflow, the burial prevents the two structural cysteines also from being oxidized to a stabilizing disulfide bond. Indeed, PDIs act *in vivo* probably already during the translocation of the still fully unfolded VHH polypeptide through the plasma membrane (of E. *coli*) or the ER membrane of a eukaryote and thus before the VHH adopts its fold. Likewise, the textbook example for measuring PDI activity utilized denatured folding substrates, such as RNase A with scrambled (i.e. incorrectly formed) disulfide bonds (Goldberger *et al.,* 1963) (Tu and Weissman, 2004). A VHH (such as the here described anti-Nup VHHs) produced under reducing conditions is, however, a correctly folded entity, because it can bind its target perfectly well (see Pleiner *et al.,* 2015 and below, Figure 11). Indeed, this poses the question if and how a chaperone/ folding enzyme can recognize such a reduced VHH as a folding substrate.

The inventors used oxidized glutathione (GSSG) as an oxidant and the three (periplasmic) PDIs from *E*. *coli,* namely DsbA, DsbC, and DsbG, as catalytic enzymes. In each case, a given VHH was incubated with various combinations of GSSG and the above-mentioned enzymes. Disulfide bond formation was then assessed by non-reducing SDS PAGE, where the (more compact) disulfide-bridged form runs faster than the reduced form (see Figures 4, 5, 6, 8C, 9, 10, 14).

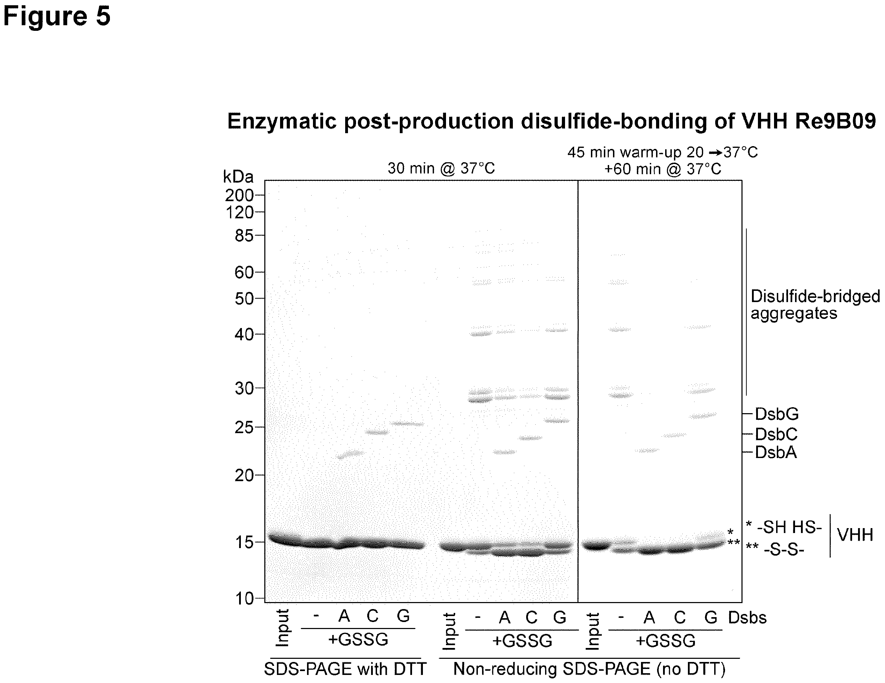

Re9B09, an anti-SARS-CoV-2 VHH antibody (SEQ ID NO: 44) (Güttler *et al.,* 2021), was a first example for a post-production disulfide-bonding (Figure 5). Incubations were performed at 37°C because this corresponds to the body temperature of alpacas (from which the VHH originated) and because this is the optimal growth temperature of E. coli and should therefore be an ideal temperature for the action of the Dsb enzymes that are of E. coli origin. Incubation with GSSG alone (2 mM) resulted already in some (10-20%) intramolecular disulfide bond formation, however, higher molecular weight bands were also prominent, pointing to intermolecular S-S bridges and thus undesired aggregates (Figure 5). Consistent with this, the mixture turned visibly cloudy indicating aggregation. The presence of DsbA or DsbC (1.5 µM) increased the yield of VHH with an intramolecular disulfide bond to ~60-70% but still, aggregates and precipitating reduced VHH were still prominent. The solution to the problem was to adapt the temperature regime of the incubation to the properties of this VHH. Specifically, this included a slow warmup phase (over 45 minutes) from 20°C to 37°C. Under these conditions, either DsbA or DsbC (1.5 µM) suppressed aggregation completely and conferred a complete intramolecular disulfide bonding (Figure 5).

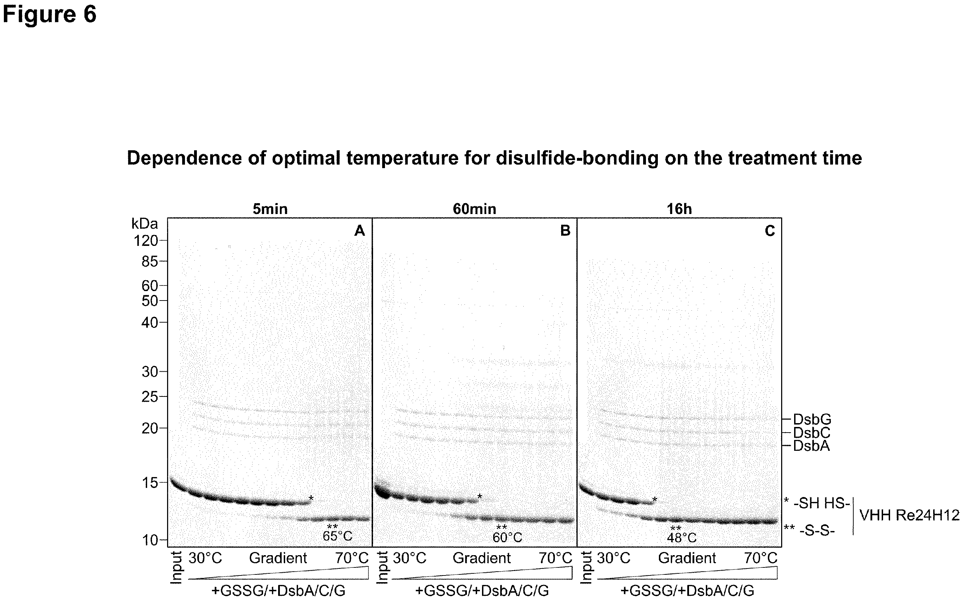

Re24H12, another anti-SARS-CoV-2 VHH antibody (SEQ ID NO: 45) showed no disulfide bond formation when tested under the same conditions (37°C), even when all three Dsb enzyme had been added. Surprisingly, however, a quantitative disulfide bond formation was observed when the treatment temperature was raised to 65°C and kept for 5 minutes (Figure 6A). The optimal treatment temperature was here determined by pipetting a master mix (35 µM VHH, 2 mM GSSG, and 1.5 µM each DsbA, DsbC, and DsbG), aliquoting this to PCR tubes, incubating the 12 parallel samples in a gradient-thermocycler with end-temperatures reaching a range between 30°C and 70°C, and analyzing disulfide-bonding in each of the samples by non-reducing SDS-PAGE.

65°C is a rather non-physiological temperature, lethal for either E. coli or mammalian cells, but the effective treatment at this temperature is explainable by the assumption that thermal unfolding of the VHH is the first and essential step in the reaction. Such unfolding would transiently make one of the two buried thiol groups accessible and thus allow its oxidation, through a covalent (SS) intermediate with either a Dsb or a glutathione molecule, followed by the displacement of the enzyme/ glutathione by the thiol group of the second VHH cysteine.

If unfolding were indeed the rate-limiting step, then one would predict that slower unfolding at lower temperature can be compensated by longer incubation times. Indeed, the optimal temperature dropped to 60° and 48°C when the treatment was extended to 60 minutes or 16 hours, respectively (Figure 6B-C).

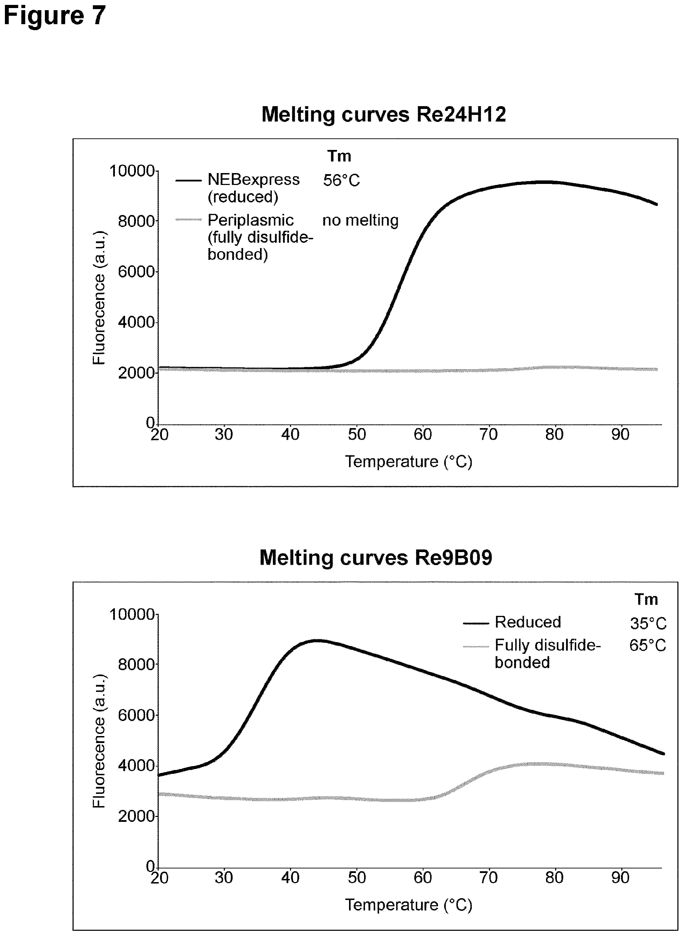

Thermal unfolding can be measured by differential scanning fluorimetry or DSF for short (also called thermofluor), using, e.g., SYBR orange as an indicator. Such analysis revealed that fully disulfide-bonded Re24H12 is hyperthermostable, resisting 95°C (Figure 7). Its reduced form had a nominal melting temperature (Tm) of 58°C (as defined by the inflection point of the melting curve). This corresponds approximately to a half-maximal unfolding and is close to the optimal disulfide-bonding temperature in the short incubation setup. The onset of unfolding is seen at ~50°C, which in turn is close to the optimal disulfide bonding temperature of 48°C in the 16 h incubation.

Interestingly, the reduced VHH Re9B09 has a rather low Tm of 35°C, explaining why an endpoint temperature 37°C was already sufficient for a quantitative post-production disulfide bond formation in this particular VHH. The low Tm also explains why the slow warmup regime was here so beneficial. It avoids an initially high rate of thermal unfolding, thus a high concentration of aggregation-prone intermediates and thus aggregation in the multi-molecular aggregation reaction.

The inventors analyzed KG1D08 in the same way, which is a VHH with particularly good NPC-staining properties (Figures 1 and 2). Its reduced form has a melting temperature (Tm) of 46°C (Figure 8A and C) and showed quantitative disulfide bond formation when incubated for 5 minutes at a final temperature of 52°C, reached during a 45 minute warmup phase (Figure 9). Pre-trials had also here established that such slow warming and thus an initially slow melting makes the reaction more robust, probably because a low concentration of melted intermediates makes them less likely to aggregate with each other. The Dsb enzymes/ glutathione can trap the unfolding intermediates as soon as they appear, catalyze disulfide bond formation, which is then irreversible under these conditions, because the disulfide-bonded form has a ≥20°C higher melting temperature and cannot participate in the back-reaction.

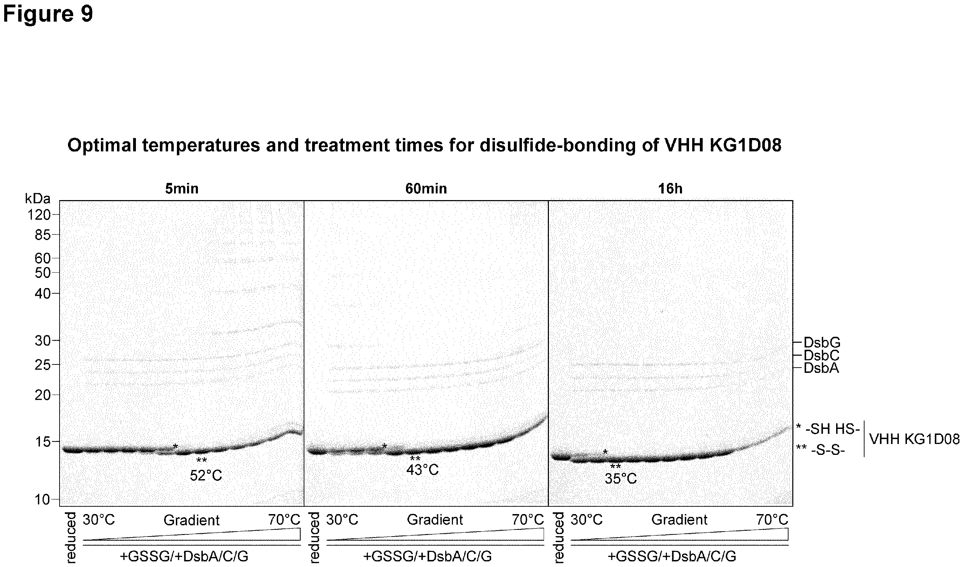

Consistent with this interpretation, the inventors found that too high incubation temperatures favored the formation of higher order aggregates (Figure 9). An upper threshold is here the onset of melting of the disulfide-bonded form (which is 10-15°C below the nominal Tm). An incubation temperature near or above the nominal Tm of the reduced form is advisable only in the slow warmup-regime.

Extending the incubations at the final temperatures, from 5 to 60 minutes or to 16 hours, lowered the optimal temperature for disulfide bonding in KG1D08 from 53°C to 43°C and 35°C, respectively. These lower temperatures coincide, again, with the onset of melting of the reduced KG1D08 (see Figure 8C).

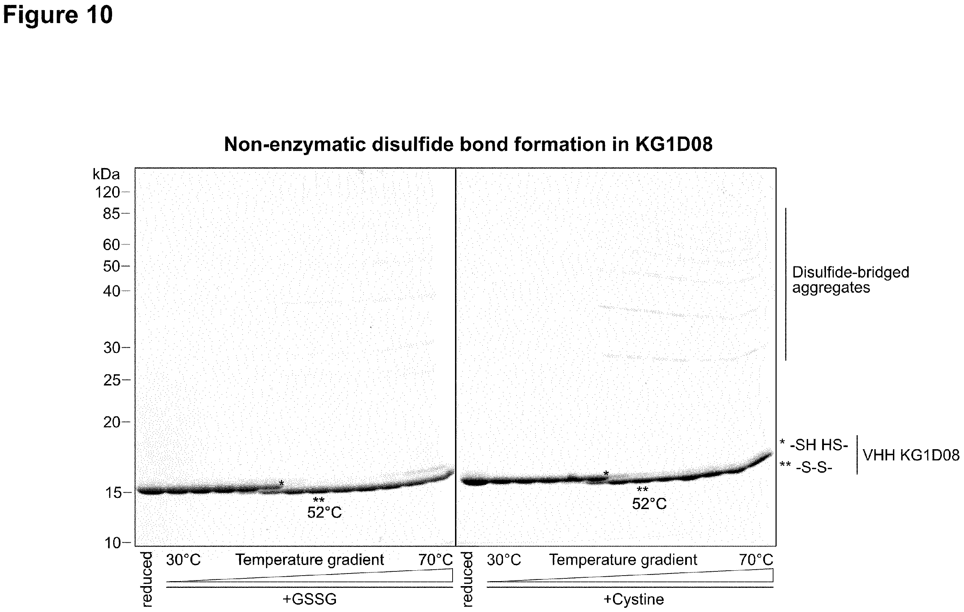

Quite unexpectedly, the disulfide bond formation in KG1 D08 did not require an addition of any PDI (Figure 10). The presence of GSSG as an oxidant was sufficient, and even the temperature optimum (52°C) of the reaction did not change when the enzymes were omitted. GSSG was, however, superior to cystine as an oxidant, leading to less aggregates and a more complete formation of the intramolecular disulfide bond (Figure 10). This can be explained by a higher hydrophilicity of a covalent intermediate with glutathione as compared to a cysteinylated species and thus a lower probability of aggregation. In any case, either treatment (with or without enzyme) allows for complete disulfide bonding and is thus far superior to the alternative of VHH expression in *E*. *coli* Shuffle, which yielded only 10-20% disulfide-bonded KG1D08 (Figure 8C).

The treatment of a VHH at elevated temperatures might appear harsh. Nevertheless, it does not compromise function. KG1D08 with post-production disulfide-bonding bound its target equally well as the initially reduced form, with a KD of ~2 nM to the human Nup133 β-propeller and with a low picomolar KD to the Xenopus antigen (Figure 11).

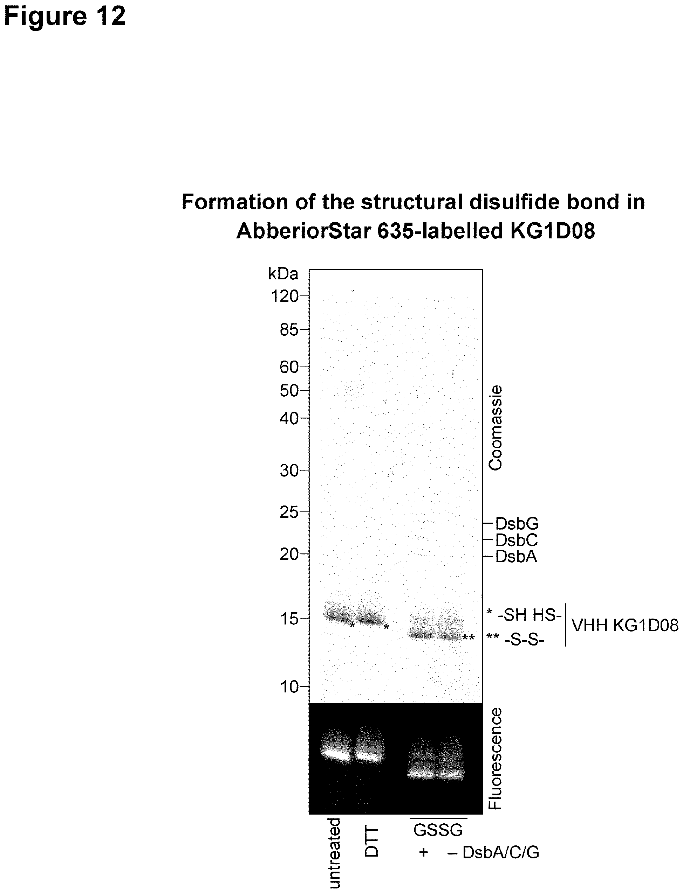

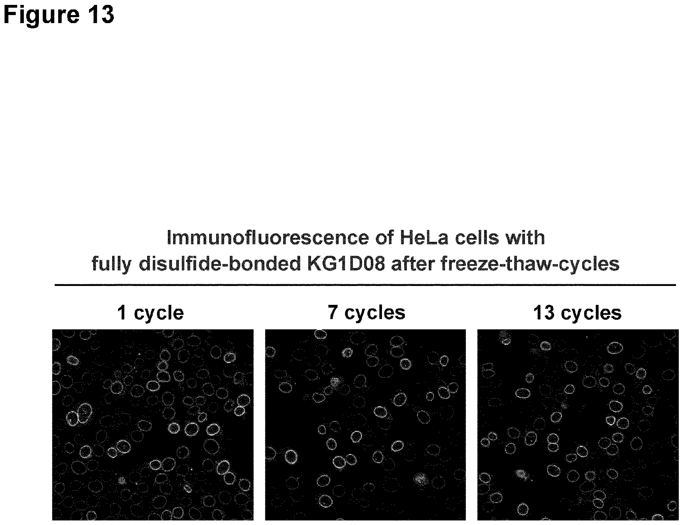

The inventors developed KG1D08 for imaging applications, expressed it under reducing conditions with ectopic cysteines, which in turn were modified with fluorophore-maleimides. This procedure left the structural cysteines non-modified but reduced. The conjugate performed very well in imaging (Figure 1 and 2) but was instable and sensitive to freeze-thaw cycles (Figure 3). A post-production, post-labelling disulfide bonding in the labelled VHH (Figure 12) solved this problem completely and yielded an imaging reagent that performed very well in immunofluorescence even after numerous freeze-thaw cycles (Figure 13).

A final example is Nb116 (SEQ ID NO: 40) - a secondary VHH directed against the anti-rabbit IgG Fc fragment (Table 1). In labelled form, it allows, for example, to use primary rabbit anti-Nup antibodies for imaging NPCs. When expressed in NEB Shuffle, this VHH can reach up to 50% disulfide-bonding. A post-production treatment at 60°C allows for a quantitative conversion into a hyperthermostable, disulfide-bridged form. Thus, the treatment strategy disclosed here allows for an excellent stabilization of fluorophore-labeled VHHs that initially had a reduced structural disulfide bond. It is a generally applicable procedure, requiring only GSSG (or a similar oxidant), a matching of the incubation temperature to the melting temperature of the VHH, and in some cases the help of PDI enzymes, such as DsbA or DsbC from *Escherichia coli* that are remarkably resistant against thermal denaturation.

The VHH antibodies may be used for imaging applications, e.g., confocal laser scanning microscopy or stimulated emission depletion (STED) microscopy. They were found to be cross-reactive with NPCs from the frog Xenopus laevis. For staining, two or more, e.g., three VHHs labelled with different labelling agents such as fluorophores may be combined, demonstrating their utility in multi-color co-localizations.

In some embodiments, VHH antibodies are combined into sets comprising a plurality of VHH antibodies.

Further, the inventors noted that the stability, e.g., the thermostability of VHH antibodies may be improved by providing VHH antibodies that are quantitatively oxidized and form an intramolecular structural disulfide bond between the two structural cysteines in the VHH sequence.

### VHH antibodies

The present invention relates to a VHH antibody, which is a monovalent heavy chain-only antibody comprising a CDR1 domain, a CDR2 domain and a CDR3 domain linked by framework regions including, but not being limited to, whole VHH antibodies, e.g. native VHH antibodies comprising framework regions derived from camelids, and modified VHH antibodies comprising modified framework regions, VHH antibody fragments and VHH antibody fusion proteins, e.g. a fusion protein with an immunoglobulin or non-immunoglobulin peptide or polypeptide, as long as it shows the properties according to the invention.

The present invention is also directed to a covalent or non-covalent conjugate of a VHH antibody molecule to a non-proteinaceous structure, for example, a labelling agent, a capture group such a solid phase-binding group, or an effector group such as a toxin. For example, the heterologous moiety may be from a fluorescence group, biotin, an enzyme such as a peroxidase, phosphatase, or luciferase, a hapten, an affinity tag, or a nucleic acid such as an oligonucleotide.

The VHH antibody of the present invention is particularly a monoclonal VHH antibody characterized by a specific amino acid sequence. The VHH antibody may be produced in a prokaryotic host cell, a yeast cell or a mammalian cell. In certain embodiments, the VHH antibody is non-glycosylated. In certain embodiments, the VHH antibody is glycosylated, wherein a carbohydrate structure may be derived from a glycosylation site introduced into the VHH sequence and/or from a fusion partner.

A VHH antibody according to the present invention is characterized by (i) a CDR3 sequence, (ii) a combination of a CDR1 sequence, a CDR2 sequence, and a CDR3 sequence, (iii) by a complete VHH sequence, or (iv) by competition with a specific reference antibody. Specific CDR and VHH sequences are provided in the Tables, Figures and the Sequence Listing. According to the present invention, sequences related to the above sequences are encompassed. These related sequences are defined by having a minimum identity to a specifically indicated amino acid sequence, e.g., a CDR or VHH sequence. This identity is indicated over the whole length of the respective reference sequence and may be determined by using well-known algorithms such as BLAST.

In particular embodiments, a related CDR3 sequence has an identity of at least 80% or at least 90% or at least 95% to a specifically indicated CDR3 sequence, e.g., a substitution of 1, 2, or 3 amino acids.

In particular embodiments, a related combination of a CDR1 sequence, a CDR2 sequence, and a CDR3 sequence has an identity of at least 80% or at least 90% or at least 95% to a specifically indicated combination of a CDR1 sequence, a CDR2 sequence, and a CDR3 sequence, e.g., a substitution of 1, 2, 3, 4, 5 or 6 amino acids by different amino acids.

In particular embodiments, a related VHH sequence has an identity of least 70%, at least 80%, at least 90%, at least 95% or at least 98% to a VHH sequence, e.g., a substitution of 1, 2, 3, 4, 5 or up to 20 amino acids.

Further, the present invention relates to a nucleic acid molecule, e.g., a DNA molecule, encoding a VHH as indicated above, or a vector, comprising said nucleic acid molecule as indicated above in operative linkage with an expression control sequence, particularly with a heterologous expression control sequence. Furthermore, the invention relates to a cell comprising a nucleic acid molecule or a vector as described above. Vectors for the recombinant production of VHH antibodies are well-known in the art. In certain embodiments, the vector is an extrachromosomal vector. In other embodiments, the vector is a vector for genomic integration. The cell may be a known host cell for producing antibodies or antibody fragments, e.g., a prokaryotic cell such as an E. coli or a Bacillus sp. cell, a yeast cell, particularly a Pichia yeast cell, an insect cell, or a mammalian cell, e.g., a CHO cell, or a plant cell. In certain embodiments, the cell comprises the nucleic acid or the vector extrachromosomally. In other embodiments, the cell comprises the nucleic acid or the vector integrated into the genome, e.g., as a genomically integrated expression cassette.

A particular aim of the invention has been to generate stable VHH antibodies useful for imaging mammalian NPCs. The inventors prepared VHH antibodies specifically directed against a component of a mammalian nuclear pore complex (NPC) selected from the group comprising Nup133, Nup358, Nup93, Nup35, and the Nup214·Nup88·Nup62 heterotrimer. In particular embodiments, the VHH antibodies are specifically directed against a component of a human nuclear pore complex (NPC) selected from the group comprising human Nup133, human Nup358, human Nup93, human Nup35, and the human Nup214·Nup88·Nup62 heterotrimer.

In some embodiments, the component of the human NPC is selected from human Nup133 (UniProt #ID: Q8WUM0), human Nup358 (UniProt #ID: P49792), human Nup93 (UniProt #ID: Q8N1F7), human Nup35, (UniProt #ID:Q8NFH5), and the human Nup214 (UniProt #ID: P35658)·Nup88 (UniProt #ID: Q99567) Nup62(UniProt #ID: P37198) heterotrimer.

Specific embodiments of such VHH antibodies are disclosed herein.

A further aim of the invention has been to generate stable VHH antibodies against the Fc region of an antibody, e.g., a rabbit IgG antibody useful as secondary detection reagent in imaging applications.

A specific embodiment of such VHH antibodies is disclosed herein.

The antibodies were produced recombinantly and modified with labelling agents, e.g., fluorophores, which were attached to solvent-accessible ectopic cysteines introduced into the antibody sequence. The term "ectopic" means that the cysteine has been added to the sequence and was not present in the original VHH sequence. The term "solvent-accessible" means the cysteine is accessible to the surrounding medium and thus to the attachment of a labelling agent when the VHH antibody is in a folded state. In some embodiments, the VHH antibody comprises 1, 2, 3, or 4 to solvent-accessible ectopic cysteines. A solvent-accessible ectopic cysteine residue may be located in the N-and/or C-terminal region and/or in the folded part of the VHH antibody, avoiding the paratope regions.

In particular embodiments, the VHH antibody comprises at least one heterologous cysteine-containing amino acid sequence at the N-terminus and/or at the C-terminus of the VHH sequence. The heterologous cysteine-containing amino acid sequence typically has a length of up to about 20 amino acids, e.g., about 1 to about 20 amino acids, or about 4 to about 10 amino acids.

In some embodiments, the heterologous amino acid sequence comprises one cysteine. A cysteine present in the heterologous amino acid sequence is an ectopic cysteine, particularly a solvent-accessible ectopic cysteine. In some embodiments, the heterologous cysteine-containing amino acid sequence is flexible and/or comprises hydrophilic amino acids, e.g., amino acids selected from Gly, Ser, Thr, Gln, Asn, Glu, and Asp, particularly from Gly, Ser, Thr and Glu.

In particular embodiments, the VHH antibody comprises a heterologous cysteine-containing amino acid sequence at the N-terminus and a heterologous cysteine-containing amino acid sequence at the C-terminus of the VHH sequence.

In some embodiments, the VHH antibody further comprises at least one heterologous tag-containing amino acid sequence, e.g., for purification. In some embodiments, the tag-containing amino acid sequence is a poly-His sequence comprising up to about 20 histidine residues or even more, e.g., a His-SUMO or a His-NEDD8 tag. In some embodiments, the tag-containing amino acid sequence is cleavable, e.g., by a protease under conditions where the residual VHH antibody remains intact.

In some embodiments, the heterologous amino acid sequence comprises a heterologous tag-containing amino acid sequence, particularly a cleavable tag-containing amino acid sequence and at least one heterologous cysteine-containing amino acid sequence.

In some embodiments, the VHH antibody carries a labelling agent. The labelling agent may be any compound to be known in the art for labelling, e.g., detecting and/or immobilizing a polypeptide. Examples of labelling agents are fluorophores, biotin, DNA oligonucleotides, enzymes, electron spin resonance (EPR) probes, or groups for immobilization to a chromatographic support. The labelling agent is preferably attached to a solvent-accessible ectopic SH group on the VHH antibody.

The labelling agent may be attached to the VHH antibody at as a thiol-reactive compound, e.g., an maleimide-, iodoacetamide-, bromoacetamide- or acrylamide-activated compound to a solvent-accessible ectopic SH group on the VHH antibody.

### Stability

For the intended applications, a VHH antibody has survive long-term storage and continued use under various conditions: at 4°C, freeze-thaw cycles, changes in temperature without a loss of activity or aggregation.

A good predictor for stability is thermostability, which can be measured, e.g., by thermal shift assays or specifically by differential scanning fluorimetry.

In a particular embodiment, the invention relates to a VHH antibody, which is stable, particularly thermostable. Preferably, the VHH antibody has a melting point (melting temperature) of at least about 65°C, of at least about 80°C, of at least 90°C or of at least about 95°C, and/or an aggregation temperature of at least about 50°C, of at least about 60°C, of at least 70°C or of at least about 80°C when measured under non-reducing conditions. Melting and aggregation temperatures are determined as described herein.

In particular embodiments, the invention relates to a stable VHH antibody that is thermostabilized which comprises two structural cysteines that are quantitatively oxidized and form an intramolecular disulfide bond. The term "quantitatively" means that the oxidized form with an intramolecular disulfide bond is present in a VHH antibody preparation in an amount of at least about 85%, at least 90% or at least about 95% as measured by non-reducing gel electrophoresis, as shown in the figures.

### Sets of VHH antibodies

In a further aspect, the present invention relates to a set comprising at least 2, 3, 4 or more of the above VHH antibodies. In such a set, the individual VHH antibodies are present in suitable molar ratios. Typically, the molar ratios are in the range of about 2:1 to about 1:2, particularly about 1.5:1 to about 1:1.5, even more particularly about 1:1. In certain embodiments, the VHH antibody set may comprise a single composition wherein the VHH antibodies in said set consist of a predetermined number of different species of VHH antibodies as described above. The VHH antibody set may comprise a plurality of compositions each comprising a different species of VHH antibody as described above. The set of the present invention may be free from other VHH antibodies.

In some embodiments, the set of VHH antibodies comprises at least 2, e.g., 2, 3, 4, or 5 VHH antibodies each recognizing a different component of a mammalian, e.g., human nuclear pore complex (NPC) selected from the group comprising Nup133, Nup358, Nup93, Nup35, and the Nup214·Nup88·Nup62 heterotrimer.

In some embodiments, the set of VHH antibodies comprises at least 2, e.g., 2, 3, 4, or 5 VHH antibodies selected from:
- a VHH antibody recognizing Nup133;
- a VHH antibody recognizing Nup358;
- a VHH antibody recognizing Nup93;
- a VHH antibody recognizing Nup35; and
- a VHH antibody recognizing the Nup214·Nup88·Nup62 heterotrimer.

In specific embodiments, the set comprises VHH antibodies selected from.
- a VHH antibody recognizing Nup133, a VHH antibody recognizing Nup358, a VHH antibody recognizing Nup93, a VHH antibody recognizing Nup35; and a VHH antibody recognizing the Nup214·Nup88·Nup62 heterotrimer;
- a VHH antibody recognizing Nup133 and a VHH antibody recognizing Nup93;
- a VHH antibody recognizing Nup358 and a VHH antibody recognizing Nup35;
- a VHH antibody recognizing Nup133 and a VHH antibody recognizing the Nup214·Nup88·Nup62 heterotrimer;
- a VHH antibody recognizing Nup133, a VHH antibody recognizing Nup358, and a VHH antibody recognizing Nup35;
- a VHH antibody recognizing Nup133, a VHH antibody recognizing Nup358, and a VHH antibody recognizing Nup93.

In some embodiments, VHH antibodies in a set may be conjugated to different labelling agents, e.g., compatible fluorophores of different spectral properties such as excitation and/or emission properties. These sets may be used to generate 2D and 3D multicolor nanoscopic patterns that can be used for benchmarking super-resolution fluorescence microscopes. Alternatively, individual VHHs can also be combined when carrying the same or spectrally similar labelling agents, e.g., fluorophores, in order to obtain an additive, stronger signal.

### Production of VHH antibodies

VHH antibodies may be produced by methods well known in the art.

A VHH antibody may be recombinantly produced in a suitable host cell, e.g., in a prokaryotic or eukaryotic host cell or host organism. For this purpose, a nucleic acid molecule encoding the VHH antibody is introduced into the host cell or host organism and expressed in the host cell or host organism. The VHH antibody may be obtained from the host cell or the culture medium according to known procedures.

In some embodiments, the VHH antibody may be produced in bacteria or yeast, e.g., *Pichia pastoris, Saccharomyces cerevisiae,* or *Hansenula polymorpha,* may be preferred.

In some embodiments, the VHH antibody is recombinantly produced in a bacterium, e.g., *E. coli* or *Bacillus.* For example, expression in a bacterium may involve cytoplasmic and/or periplasmic expression and purification of the VHH antibody from the host cell, or secretory expression and purification of the VHH antibody from the culture medium. In certain embodiments, the nucleic acid sequence encoding the VHH antibody is fused to at least one sequence directing the expression to the periplasm and/or into the culture medium.

In particular embodiments, the present invention relates to a novel method for producing a stable VHH antibody. This method is not only applicable to the anti-NPC VHH antibodies as described herein but for any type of VHH antibody comprising two structural cysteines capable of forming an intramolecular disulfide bond. For example, this method is also applicable to anti-SARS-CoV2 VHH antibodies, e.g., VHH antibodies Re9B09 (SEQ ID NO: 44) and Re24H12 (SEQ ID NO: 45) as shown in the Figures and Examples. This method is specifically applicable to a VHH antibody comprising two structural cysteines and further at least one solvent-accessible ectopic cysteine as described herein.

The inventors found that the structural disulfide bond of a VHH is important for its stability and that this disulfide bond can be introduced in a workflow that includes (i) expression of a VHH antibody optionally with a solvent-accessible ectopic cysteine for later labeling, under reducing conditions, e.g., cytoplasmic expression in a normal *E. coli,* or semi-reducing conditions, e.g., cytoplasmic expression in *E. coli* NEB Shuffle, (ii) VHH purification including a final reduction step to ensure an -SH state of the ectopic cysteines, (iii) modification of the ectopic (and fully exposed) cysteines with a labelling agent, e.g., a thiol-reactive labelling agent, while keeping the structural cysteines unmodified, (iv) a post-treatment in the presence of an oxidant that converts the two structural cysteines (which are buried in the hydrophobic core of the VHH) to a disulfide bond, and (v) obtaining the resulting thermostabilized VHH antibody.

Thus, a further aspect of the invention is a novel method of producing a stable VHH antibody comprising the steps:
(i) expressing a VHH antibody comprising two structural cysteines and optionally at least one solvent-accessible ectopic cysteine in a host cell under reducing or at least partially reducing conditions,
(ii) purifying the VHH antibody from the host cell or the culture medium under conditions wherein the at least one solvent-accessible ectopic cysteine is in the reduced SH state;
(iii) optionally attaching a labelling agent to the at least one solvent-accessible ectopic cysteine under conditions wherein the structural cysteines are kept unmodified,
(iv) subjecting the VHH antibody to an oxidation that converts the two structural cysteines to a disulfide bond, and
(v) obtaining a thermostable VHH antibody wherein the two structural cysteines are quantitatively oxidized to a disulfide bond.

In some embodiments, the VHH antibody comprises a solvent-accessible ectoptic cysteine as herein descrbed above, e.g., in its N-and/or C-terminal region and/or in its folded part, avoiding the paratope regions.

In some embodiments, the VHH antibody comprises at least one heterologous solvent-accessible ectopic cysteine-containing amino acid sequence at its N- and/or C-terminus, particularly a heterologous solvent-accessible ectopic cysteine-containing amino acid sequence having a length of up to about 20 amino acids.

In some embodiments, the VHH antibody further comprises at least one heterologous tag-containing amino acid sequence as herein described above.

The antibody expression in the host cell according to step (i) is carried out under reducing or at least partially reducing conditions. These conditions may comprise expression of the VHH antibody in the cytoplasm of a prokaryotic host cell such as *E.coli* or a eukaryotic host cell such as a yeast, protozoan, or animal cell optionally followed by a reduction.

The antibody purification in step (ii) may comprise a treatment with a reducing agent such as a thiol, e.g., DTT (dithiothreitol) or a phosphine, e.g., TCEP (tris(carboxyethyl)phosphine), followed by a removal of the reducing agent prior to an optional subsequent incubation with the labelling agent. In some embodiments, step (ii) comprises a chromatographic purification in the presence of a reducing agent.

In some embodiments, the labelling agent is a fluorophore, biotin, a DNA oligoucleotide or another labelling agent as herein desscribed above.

In some embodiments, the labelling agent is a thiol-reactive compound as herein described above, e.g., a maleimide-activated compound.

Step (iv) comprises a treatment of the VHH antibody with an oxidant. In some embodiments, a disulfide compound such as oxidized glutathione (GSSG), cystine, dithioglycolic acid or 2,2'dithiopyridine may be used as an oxidant.

In some embodiments, step (iv) is carried out in the presence of a PDI such as DsbA from E. coli (SEQ ID NO: 41), DsbC from E. coli (SEQ ID NO: 42), or DsbG from E. coli (SEQ ID NO: 43).

Further, the inventors found the oxidation is preferably carried out under conditions wherein an at least partial thermal unfolding of the VHH antibody takes place. In this stage, the VHH antibody comprises reduced structural cysteines.

Thus, in some embodiments, the VHH antibody is subjected to an incubation at a temperature and for a time period wherein a transient (reversible) thermal unfolding of the VHH antibody is promoted. The incubation temperature is preferably in the range between the nominal melting temperature (Tm) of the reduced form of the VHH antibody (with reduced structural cysteines) and the onset of melting of the reduced form of the VHH antibody (which is typically 10°C-15°C below Tm). An incubation temperature above the onset of melting of the reduced form is advisable only in a slow warmup-regime. An upper temperature threshold is the onset of melting of the disulfide-bonded form (which is typically 10-15°C below the nominal Tm of the disulfide-bonded form).

The melting temperatures of individual VHH antibodies may vary considerably. The optimal incubation temperature of an individual VHH antibody may be determined as described in the Figures and Examples, particularly by the methods disclosed in Figures 6, 9, 10, and 14.

The incubation during the oxidation step may involve a slow warm-up regime, e.g., involving a temperature increase of about 1°C per min or less over a temperature range of at least 10°C, preferably of about 15°C.

The incubation time of the oxidation step can vary to great extent, e.g., between about 5 min and about 24 h, preferably between 1 h and 16 h. Typically, the incubation time needed for completing reaction correlates inversely with the incubation temperature.

Further, the invention shall be explained in more detail by the following Figures and Examples.

### Figures

**Figure 1****: Anti-nuclear pore complex (NPC) VHH antibodies and their application in confocal laser scanning microscopy.** VHH antibodies were generated by immunizing alpacas with human as well as Xenopus antigens, constructing immune libraries from blood samples, and phage display using folded nucleoporin (Nup) domains as baits. Antigens included the N-terminal α-helical domain of Nup358 (residues 1-145), the RRM domain of Nup35, the of Nup93, the β-propeller of Nup133, and the heterotrimeric coiled-coil of the Nup214·Nup88·Nup62 complex (or Nup214 complex for short). VHH antibodies were expressed in E. coli NEB Shuffle with two ectopic cysteines each (placed on N- and C-termini). These ectopic cysteines were kept reduced during purification and were modified with the fluorophore Alexa647 maleimide.
   (**A)** For immunofluorescence, HeLa-K cells were grown on 10-well glass slides, fixed with 2.4% paraformaldehyde for 5 minutes at room temperature (RT), and permeabilized with 0.3% TritonX100 in PBS. Cells were then blocked with 1% (w/v) bovine serum albumin (BSA) for 30 mins and stained with 35 nM of the indicated Alexa 647-labeled tracking nanobodies for 30 mins at RT. Finally, the excess of tracking nanobodies was washed off and cells were imaged using a Leica SP8 confocal microscope (Leica, Germany) and excitation by the 638 nm laser line. The figure shows confocal sections through the equator of the cell nuclei. The pattern of a crisp nuclear rim is typical for a nuclear pore complex (NPC) stain. Scale bar, 10 µm.
   **(B)** Cell nuclei were assembled from *Xenopus* egg extract and sperm chromatin as an assembly template. They were then fixed with paraformaldehyde (PFA), spun onto coverslips and stained with the indicated mix of three different VHH antibodies labeled with Alexa488, Alexa568 and Alexa647-maleimide. Confocal scans were recorded sequentially. Scale bar, 5 µm (upper panels, equatorial scan) and 1 µm (lower panels, scan of nuclear surface).
**Figure 2****: Application of anti-NPC VHHs in super-resolution microscopy.** Immunofluorescence was performed as described in Figure 1 with the following modifications: AbberiorStar 635 maleimide was used for labeling, cells were mounted in SlowFade Gold (Thermo Fisher Scientific) imaging medium to protect the dyes from photobleaching, images were acquired with a STEDycon microscope (Abberior Instruments) using the 640 nm laser and deconvoluted using the Huygens Professional software (version 19.10) with a saturation factor of 80 and an immunity factor of 1. Images reveal the typical ring-like NPC structure originating from their 8-fold rotational symmetry. Scale bar, 500 nm.
**Figure 3****: Sensitivity of cytoplasmically expressed VHH KG1D08 to repeated freeze-thaw cycles.** Immunofluorescence of HeLa cells was performed similarly to Figure 1, but with a few modifications. Cells were first semi-permeabilized with 30 µg/ml digitonin in 'transport buffer' (20 mM HEPES/ KOH pH 7.5, 3.5 mM MgAc, 110 mM KAc, 1 mM EGTA, 250 mM sucrose) for 5 minutes at room temperature. They were then incubated with 40 nM anti-Nup133 VHH KG1D08 expressed in NEB Shuffle and labeled with AbberiorStar 635. On the left, the VHH was frozen once (as a 20µM stock in PBS plus 20% glycerol) in liquid nitrogen and thawed at room temperature immediately before the experiment. On the right side, the VHH was frozen and thawed six more times after a 4-fold dilution in transport buffer. Note that the repeated freezing and thawing caused a severe loss of staining activity. This loss is probably due to the irreversible denaturation of KG1D08 molecules that were not stabilized by the structural disulfide bond. The low residual activity is probably due to a small (-10-20%) fraction of disulfide-bonded VHH formed during expression in NEB Shuffle (see below, figure 8C).
**Figure 4****: Disulfide bonding state of VHHs dependent on the mode of expression in** ***Escherichia coli**.* VHH antibody Re24H12 (directed against the receptor binding domain of SARS-CoV-2) was expressed in three ways: in the reducing cytosol of NEB Express, the partially oxidizing cytosol of NEB Shuffle, and under the oxidizing conditions of the periplasm. The disulfide bonding of these preparations was then elucidated by SDS polyacrylamide electrophoresis (SDS PAGE) under non-reducing conditions (keeping any formed disulfide bonds intact), each in comparison to a sample prior reduced by dithiothreitol (DTT). Gel-staining was performed with Coomassie Brilliant Blue G250. The disulfide bond-containing VHH (**, lower band) migrates faster than the corresponding reduced form (*, upper band). The analysis revealed that cytoplasmic production in NEB Express gave a VHH with fully reduced cysteines, while cytoplasmic expression in Shuffle resulted in ~30% bond formation. Only the periplasmically expressed species was quantitively disulfide-bonded.
**Figure 5****: Enzymatic post-production disulfide bond formation.** The anti-SARS-CoV-2 VHH antibody Re9B09 was produced cytoplasmically in NEB Express as described in Figure 4. It was thus initially fully reduced. Left: A 35 µM VHH stock in Dsb buffer (50 mM Tris/ HCl pH 8.0, 150 mM NaCl) was then incubated for 30 minutes at 37°C with the indicated additives, namely 2 mM oxidized glutathione (GSSG) as an oxidant, and 1.5 µM of the E. coli thiol: disulfide exchange proteins DsbA, DsbC, or DsbG (concentrations refer to the enzyme monomers). Samples were then analyzed by reducing and non-reducing SDS PAGE as described in Figure 4. The incubation with GSSG alone resulted in an incomplete formation of the structural disulfide bond as well in higher molecular weight aggregates. In the presence of DsbA or DsbC, disulfide bond formation was more complete, and less aggregates were observed. Right: incubations were as on the left side, but the incubations included a slow warm-up over 45 minutes from 20°C to 37°C and a longer (60 minutes) incubation at 37°C. This regime allowed a quantitative disulfide bond formation in the presence of DsbA or DsbC without aggregation. DsbG had a partially positive effect on disulfide bond formation and suppression of aggregation.
**Figure 6****: Treatment temperature and treatment time as key parameters for optimal post-production disulfide bond formation.** Fully reduced Re24H12 was produced by cytoplasmic expression in NEB express as described above. A mixture of 35 µM VHH, 2 mM GSSG, and 1.5 µM each of DsbA, DsbC, and DsbG was prepared in Dsb buffer and placed in 20 µl aliquots into a gradient thermocycler. Within 45 minutes, the samples were linearly heated to either 30°C, 32°C, 35°C, 39°C, 43°C, 48°C, 52°C, 57°C, 61°C, 65°C, 68°C, or 70°C. The end temperature was then kept for either 5 minutes, for one hour, or overnight (16 hours), before returning to 20°C. Samples were subsequently analyzed by non-reducing SDS PAGE for disulfide bonding. For the short treatment (5 minutes hold time), an end temperature of 65°C was required to convert VHH Re24H12 quantitatively to the disulfide-bonded form. For the medium-length treatment, 61°C was sufficient. This temperature optimum dropped to 48°C for a 16 hours-treatment.
**Figure 7****: Melting behavior of VHH Re24H12.** The VHH was prepared as indicated and as described in Figure 4. To determine the melting temperatures of the VHH in these preparations, differential scanning fluorimetry (DSF, Thermofluor) was performed, using SYPRO orange (1x, Invitrogen) as a fluorescent unfolding indicator, 20µl sample volume, and a temperature increase of 1°C every 45 seconds from 20°C to 95°C. Periplasmically expressed Re24H12 showed no melting at all, indicating hyperthermostability. The fully reduced form melted at 56°C (as defined by the inflection point of the melting curve). Note that this temperature is close to the optimal treatment temperature for disulfide bonding of this VHH in the fast-treatment protocol (5 or 60 minutes). The onset of melting occurred at ~48°C, which in turn coincides with the optimal treatment temperature in the overnight protocol.
**Figure 8****: Melting behavior of VHH KG1D08. A)** Melting analysis was as described in Figure 7. Plots of the fluorescence signals against temperature are shown. **B)** Same as A, but the first derivatives are being shown. The minima mark the respective melting temperatures (46°C for fully reduced KG1D08 and 68°C for the fully disulfide-bonded form). **C)** Analysis of disulfide bonding by non-reducing SDS-PAGE. Cytoplasmic expression in NEB Express resulted in a fully reduced VHH, cytoplasmic expression in NEB Shuffle in -10-20% disulfide bonding, and cytoplasmic expression in NEB Express followed by a treatment with GSSG (as in Figure 10) resulted in a quantitative formation of the structural disulfide bridge.
**Figure 9****: Optimal treatment temperatures for enzymatic post-production disulfide bonding of VHH KG1D08.** Optimal conditions for introducing the structural disulfide bond in the initially fully reduced KG1D08 were elucidated as described for Re24H12 in Figure 6, again in the presence of 2 mM GSSG, and 1.5µM each DsbA, DsbC, and DsbG. Note that the optimal temperature for the overnight treatment (35°) approximates the onset of melting of the fully reduced VHH, while the optimal endpoint temperature (43-52°) in the fast-treatment (5-60 minutes) approximates the nominal melting point of 46°C. The overnight treatment at lower temperature appears superior since less aggregates are formed.
**Figure 10****: Non-enzymatic disulfide bridging in KG1D08.** Left: experiment was performed as in Figure 9 (holding the endpoint temperature for 5 minutes) but without the Dsb enzymes. Note that the disulfide bond formation in this particular VHH was as efficient as in the presence of the enzymes. Right: same experiment but using cystine (oxidized cysteine) instead of GSSG as an oxidant. This had essentially the same effect but resulted in more aggregates.
**Figure 11****: Affinities of reduced and post-production disulfide-bridged KG1D08 for its Nup133 b-propeller target.** KG1D08 was expressed with two additional cysteines at its N- and C-terminus in the cytoplasm of NEB Express. After purification, these two exposed cysteines were modified with Biotin-PEG11-maleimide (Iris Biotech). Half of the preparation was treated with 2 mM GSSG, 1.5 µM each DsbA, DsbC, and DsbG for 5 minutes at 52°C with 45 minutes heat up time, resulting in quantitative disulfide bonding.
   The biotinylated VHHs were then immobilized for biolayer interferometry to High precision streptavidin sensors of an Octet Red96 machine (to a binding signal of 1 nm), using PBS+ 1% BSA as an assay buffer. Loaded sensors were then dipped for 800 seconds into 40 nM solutions of the human or the Xenopus Nup133 beta propeller to measure association, and subsequently dipped into buffer only to measure dissociation. Dissociation constants were computed using the Octet software. KG1D08 bound the human propeller with a Kd of ~2 nM, and the Xenopus propeller with a Kd probably better than 10 pM, and there was no obvious difference between the reduced and the disulfide-stabilized VHH.
**Figure 12****: Disulfide bond formation in fluorophore-labelled KG1D08.** KG1D08 was produced in fully reduced form with two additional cysteines as described above and labelled AbberiorStar 635 maleimide to a density of labelling of 2. The labelled VHH was then treated with 2 mM GSSG, 1.5 µM each DsbA, DsbC, and DsbG for 5 minutes at 52°C with 45 minutes heat up time, resulting in quantitative disulfide bonding. Analysis was by reducing/ non-reducing SDS PAGE followed by fluorescence detection and by Coomassie staining.
**Figure 13****: Resistance of fluorophore-labelled KG1D08 towards freeze-thaw cycles following stabilization with its structural disulfide bond.** Immunofluorescence was performed as in Figure 3, the only difference being that the AbberiorStar 635-labelled VHH was used after quantitative disulfide bonding as documented in Figure 12. Note that the stabilized VHH survived not only 7 but 13 freeze-thaw cycles without any loss in activity.

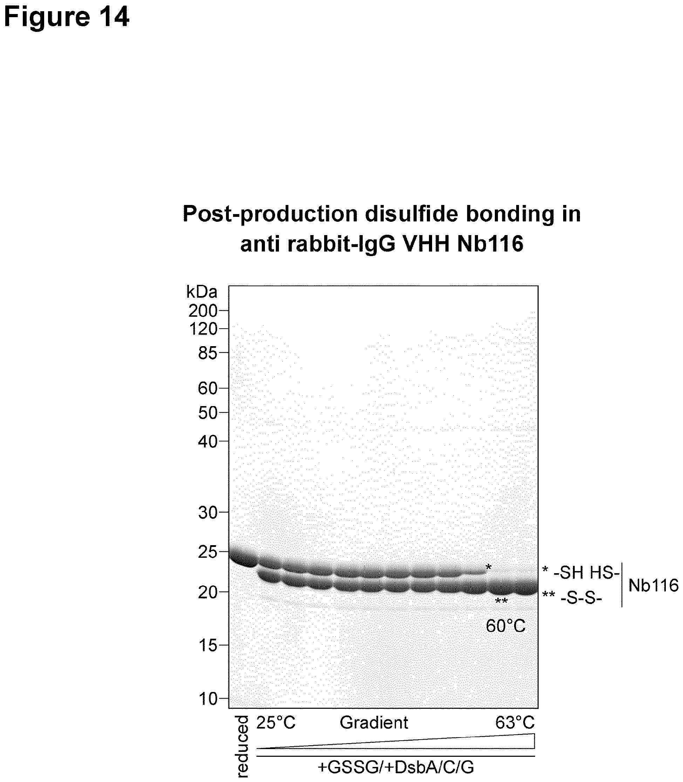

**Figure 14****: Quantitative introduction of a stabilizing disulfide bond into the anti-rabbit IgG VHH Nb116.** The VHH was produced cytoplasmically in NEB Shuffle, resulting in 50% disulfide bridge formation. This preparation was then tested for conditions allowing a quantitative disulfide bonding as described in Figures 6 and 9. 50 µM VHH was supplemented with 2 mM GSSG, and 1.5 µM each of DsbA, DsbC, and DsbG, and heated within 45 minutes from 20°C to either 25°C, 27°C, 30°C, 34°C, 38°C, 43°C, 47°C, 52°C, 56°C, 60°C, or 63°C and keeping the end temperature for 5 minutes. An end temperature of 60°C allowed quantitative disulfide bonding.

### Examples

The details for the experiment shown in Figure 1 were as follows: Cytoplasmic expression was performed either in *E.coli* NEB Express (NEB #C3037I) or NEB Shuffle (NEB #C3028J) with IPTG induction, using a plasmid encoding a His₁₄-NEDD8-VHH fusion (Pleiner *et al.,* 2015). Cell lysates were prepared by sonication and ultracentrifugation, and the fusion was bound to a Ni²⁺ chelate matrix. After extensive washing, the VHH was eluted by cleaving the N-terminal His₁₄-NEDD8 tag with bdNEDP1 protease (Frey and Görlich, 2014a, 2014b). Alternatively, Re24H12 was expressed with a cleavable N-terminal DsbA secretion signal and a C-terminal spacer-His₁₂ tag. It was purified by Ni²⁺ chelate chromatography from the periplasmic fraction (obtained by an osmotic shock). The C-terminal tag was not removed, resulting in a slower migrating species.

The three VHH preparations were then analyzed by SDS PAGE after (denaturing) alkylation of free cysteines with 50 mM iodoacetamide (supplied as a 100 mM stock in 100% formamide). This treatment leaves any formed disulfide bond intact and protects such bond against re-arrangements during sample preparation or electrophoresis. DTT-reduced control samples were obtained by denaturing reduction with 20 mM DTT + 2% SDS for one minute at 95°C, followed by alkylation with 100 mM iodoacetamide (from a 500 mM stock in water) and the addition of 100 mM Tris base (from a 2 M stock) to neutralize the released hydrogen iodide. A disulfide-bonded species shows a characteristic size shift between the two conditions on the SDS-gel.

All other experiments are illustrated by the description of the invention, figures and the corresponding legends.

### Sequences

In the following, the VHH sequences of VHH antibodies described herein (without ectopic cysteines) and PDI enzymes described herein are listed.
>KG1D08 (SEQ ID NO: 1) 
QVQLVESGGGLVQAGGSLRLSCAASGRSFRGNIMAWFRQTPGKERLFVASITGSG
YTSYENSVKGRFTISRDSARDTVDLQMTTLAPEDTAVYYCAGRFRGGHNRMPDAY
EYWGQGTQVTVSS
>KG1C05 (SEQ ID NO: 5) 
QVQLVESGGGLVQAGGSLRLACATSGILLSKAVMDWYRQIPGKQRELVARTGIRDS
ATYADFVKGRFTVSRENENNTVYLQMNSLQPEDAGLYVCKAAVTGVFGRLSEHWG
QGTQVTVSS
>KG2E04 (SEQ ID NO: 9) 
QVQLVESGGGLVQAGGSLRLSCAASGRGFSGYTWGWFRQVPGKDRTFVAAMSWI
TGSTYYADSVKGRFTISGDNAKNTLLLQMDNLKPNDTAVYYCAARSKVSPDYDIWG
QGTQVTVSS
>KG2B08 (SEQ ID NO: 13) 
QVQLVESGGGLVQAGGSLRLSCAASGRGFSGYTWGWFRQVPGKERTFVAAMSWI
TGSTYYADSVKGRFTISGDNAKNTLLLQMNNLKPGDTAVYYCAARSKVSYDYDIWG
QGTQVTVSS
>KG2B12 (SEQ ID NO: 17) 
QVQLVESGGGLVQAGGSLRLSCAASGRGFSGYTWGWFRQVPGKERTFVAAMSWI
TGSTYYADSVKGRFTISGDNAKNTLLLQMNNLKPGDTAVYYCAARSKVSYDYDIWG
QGTLVTVSS
>Re3E01(SEQ ID NO: 21) 
QVQLVESGGGLVQPGGSLRLSCAASGFTFTDYLMAWYRQAPGNQRQLVAYITSDG
STVYASSARGRFTISRDNAKNTVTLQMNSLLLDDTAVYYCSAKSGLKDYYGQGTQV
TVSS
>xhNup358-Nb2t (SEQ ID NO: 25) 
QVQLVESGGGLVQPGGSLRLSCAASGRPLSDYAMGWFRQAPGKEREFVAAISWK
GGETFYAGSVKGRFTISRDDAKNSLYLQMNNLKLEDTAEYSCAAVLGSPHYSGTHY
YPAEYDYWGQGTQVTVSS
>xhNup93-Nb3t (SEQ ID NO: 29) 
QVQLVESGGGVVQTGDSLMLSCLFSGRPGRVFGHYLMGWFRQIPGKERDVVAAIS
FSGDPTYYADSVKGRATISRDNAKNTVYLQMNGLKSEDTAVYYCAADRLPYGDSW
ASKDEYDYWGQGTQVTVSS
>xhNup35-Nb1t (SEQ ID NO: 33) 
QVQLVESGGGLVQAGGSLRLSCVGTGNISSAYTMAWYRQAAGKEREVVAGILNGG
STHYADSVKGRFTISRDNAKNTVYLQMNSLQPEDTGVYCCHVVIESTRFVYDHYW
GQGTRVTVSS
>Nb116 (SEQ ID NO: 37) 
QVQLVESGGGLVQPGGSLTLSCTVSRIINWTRMGWSRTAPGKQREFVANIDPSGTT
NYADFVKGRFAISVNNDKRTVFLQMNGLKPEDTAVYRCFTIEPDRGTNWGQGTQV
TVSS
>DsbA (SEQ ID NO: 41) 
AQYEDGKQYTTLEKPVAGAPQVLEFFSFFCPHCYQFEEVLHISDNVKKKLPEGVKM
TKYHVNFMGGDLGKDLTQAWAVAMALGVEDKVTVPLFEGVQKTQTIRSASDIRDVF
 
INAGIKGEEYDAAWNSFVVKSLVAQQEKAADVQLRGVPAMFVNGKYQLNPQGMD
TSNMDVFVQQYADTVKYLSEKK
>DsbC (SEQ ID NO: 42) 
DDAAIQQTLAKMGIKSSDIQPAPVAGMKTVLTNSGVLYITDDGKHIIQGPMYDVSGTA
PVNVTNKMLLKQLNALEKEMIVYKAPQEKHVITVFTDITCGYCHKLHEQMADYNALG
ITVRYLAFPRQGLDSDAEKEMKAIWCAKDKNKAFDDVMAGKSVAPASCDVDIADHY
ALGVQLGVSGTPAVVLSNGTLVPGYQPPKEMKEFLDEHQKMTSGK
>DsbG (SEQ ID NO: 43) 
AEELPAPVKAIEKQGITIIKTFDAPGGMKGYLGKYQDMGVTIYLTPDGKHAISGYMYN
EKGENLSNTLIEKEIYAPAGREMWQRMEQSHWLLDGKKDAPVIVYVFADPFCPYCK
QFWQQARPWVDSGKVQLRTLLVGVIKPESPATAAAILASKDPAKTWQQYEASGGKL
KLNVPANVSTEQMKVLSDNEKLMDDLGANVTPAIYYMSKENTLQQAVGLPDQKTLN
IIMGNK
>Re9B09 (SEQ ID NO: 44) 
QVQLVESGGGLVQPGGSLRLSCAASGFTLDYYAIGWFRQAPGKEREGVSRISSSD
GSTDYADSVKGRFTISRDNAKNTVYLQMNSLKPEDTAVYYCATVPGTYYSGNWYY
TWHPEAVDYWGKGTQVTVSS
>Re24H12 (SEQ ID NO: 45)

QVQLVESGGGLVQPGGSLRLSCAASGFTLDYYAIGWFRQAPGKEREGVSYIRSSD
GTTYYADSVKGRFTISRDNAKNTVYLQMNSLKPEDTAVYYCAADEAYYSELGWESP
WGWSYWGQGTQVTVSS

### References

Bardwell JC, McGovern K, Beckwith J (1991) Identification of a protein required for disulfide bond formation in vivo. Cell, 67: 581-589
Bessette PH, Åslund F, Beckwith J, Georgiou G (1999) Efficient folding of proteins with multiple disulfide bonds in the Escherichia coli cytoplasm. Proceedings of the National Academy of Sciences, 96: 13703-13708
Bulleid NJ, Freedman RB (1988) Defective co-translational formation of disulphide bonds in protein disulphide-isomerase-deficient microsomes. Nature, 335: 649-651
Edman JC, Ellis L, Blacher RW, Roth RA, Rutter WJ (1985) Sequence of protein disulphide isomerase and implications of its relationship to thioredoxin. Nature, 317: 267-270
Frey S, Görlich D (2014a) A new set of highly efficient, tag-cleaving proteases for purifying recombinant proteins. J ChromatogrA, 1337: 95-105
Frey S, Görlich D (2014b) Purification of protein complexes of defined subunit stoichiometry using a set of orthogonal, tag-cleaving proteases. J ChromatogrA, 1337: 106-115
Goldberger RF, Epstein CJ, Anfinsen CB (1963) Acceleration of Reactivation of Reduced Bovine Pancreatic Ribonuclease by a Microsomal System from Rat Liver. Journal of Biological Chemistry, 238: 628-635
Göttfert F, Pleiner T, Heine J, Westphal V, Görlich D, Sahl SJ, Hell SW (2017) Strong signal increase in STED fluorescence microscopy by imaging regions of subdiffraction extent. Proc Natl Acad Sci U S A, 114: 2125-2130
Güttler T, Aksu M, Dickmanns A, Stegmann KM, Gregor K, Rees R, Taxer W, Rymarenko O, Schünemann J, Dienemann C, Gunkel P, Mussil B, Krull J, Teichmann U, Groß U, Cordes VC, Dobbelstein M, Görlich D (2021) Neutralization of SARS-CoV-2 by highly potent, hyperthermostable, and mutation-tolerant nanobodies. EMBO J, e107985
Hamers-Casterman C, Atarhouch T, Muyldermans S, Robinson G, Hamers C, Songa EB, Bendahman N, Hamers R (1993) Naturally occurring antibodies devoid of light chains. Nature, 363: 446-448
Hampoelz B, Andres-Pons A, Kastritis P, Beck M (2019) Structure and assembly of the nuclear pore complex. Annual review of biophysics, 48: 515-536
Hatahet F, Ruddock LW (2009) Protein disulfide isomerase: a critical evaluation of its function in disulfide bond formation. Antioxid Redox Signal, 11: 2807-2850
Koenig PA, Das H, Liu H, Kümmerer BM, Gohr FN, Jenster LM, Schiffelers LDJ, Tesfamariam YM, Uchima M, Wuerth JD, Gatterdam K, Ruetalo N, Christensen MH, Fandrey CI, Normann S, Tödtmann JMP, Pritzl S, Hanke L, Boos J, Yuan M, Zhu X, Schmid-Burgk JL, Kato H, Schindler M, Wilson IA, Geyer M, Ludwig KU, Hällberg BM, Wu NC, Schmidt FI (2021) Structure-guided multivalent nanobodies block SARS-CoV-2 infection and suppress mutational escape. Science, 371: eabe6230
Lambert N, Freedman RB (1985) The latency of rat liver microsomal protein disulphide-isomerase. Biochem J, 228: 635-645
Löschberger A, van de Linde S, Dabauvalle MC, Rieger B, Heilemann M, Krohne G, Sauer M (2012) Super-resolution imaging visualizes the eightfold symmetry of gp210 proteins around the nuclear pore complex and resolves the central channel with nanometer resolution. J Cell Sci, 125: 570-575
Muyldermans S (2013) Nanobodies: natural single-domain antibodies. Annu Rev Biochem, 82: 775-797
Pleiner T, Bates M, Görlich D (2018) A toolbox of anti-mouse and anti-rabbit IgG secondary nanobodies. J Cell Biol, 217: 1143-1154
Pleiner T, Bates M, Trakhanov S, Lee CT, Schliep JE, Chug H, Böhning M, Stark H, Urlaub H, Görlich D (2015) Nanobodies: site-specific labeling for super-resolution imaging, rapid epitope-mapping and native protein complex isolation. Elife, 4: e11349
Roth RA, Mesirow ML (1984) Production and characterization of a monoclonal antibody to rat liver thiol: protein-disulfide oxidoreductase/glutathione-insulin transhydrogenase. Biochim Biophys Acta, 788: 189-192
Rothbauer U, Zolghadr K, Muyldermans S, Schepers A, Cardoso MC, Leonhardt H (2008) A versatile nanotrap for biochemical and functional studies with fluorescent fusion proteins. Mol Cell Proteomics, 7: 282-289
Sahl SJ, Hell SW, Jakobs S (2017) Fluorescence nanoscopy in cell biology. Nat Rev Mol Cell Biol, 18: 685-701
Szymborska A, de Marco A, Daigle N, Cordes VC, Briggs JA, Ellenberg J (2013) Nuclear pore scaffold structure analyzed by super-resolution microscopy and particle averaging. Science, 341: 655-658
Tu BP, Weissman JS (2004) Oxidative protein folding in eukaryotes: mechanisms and consequences. J Cell Biol, 164: 341-346
Weber M, von der Emde H, Leutenegger M, Gunkel P, Sambandan S, Khan TA, Keller-Findeisen J, Cordes VC, Hell SW (2023) MINSTED nanoscopy enters the Ångström localization range. Nat Biotechnol, 41: 569-576

## Claims

1. A set of at least 2 VHH antibodies recognizing each recognizing a different component of a mammalian nuclear pore complex (NPC) selected from the group comprising Nup133, Nup358, Nup93, Nup35, and the Nup214·Nup88·Nup62 heterotrimer.

2. The set of claim 1 wherein at least one VHH antibody comprises at least one solvent-accessible ectopic cysteine residue.

3. The set of claim 1 or 2 comprising at least one VHH antibody comprising:
(a) a CDR3 sequence as shown in SEQ. ID NO: 2, 6, 10, 14, 18, 22, 26, 30, or 34;
(b) a CDR3 sequence, which has an identity of at least 80%, at least 90% or at least 95% to a CDR3 sequence of (a); or
(c) a VHH antibody, which competes with a VHH antibody of (a) for the binding to the respective component of the NPC.

4. The set of any one of claims 1-3 comprising at least one VHH antibody comprising:
(a) a combination of CDR1, CDR2 and CDR3 sequences as shown in SEQ. ID NO: 2-4, 6-8, 10-12, 14-16, 18-20, 22-24, 26-28, 30-32, or 34-36;
(b) a combination of CDR1, CDR2 and CDR3 sequences which has an identity of at least 80%, at least 90% or at least 95% to a combination of CDR1, CDR2 and CDR3 sequences of (a); or
(c) a VHH antibody, which competes with a VHH antibody of (a) for the binding to the respective component of the NPC.

5. The set of any one of claims 1-4 comprising at least one VHH antibody comprising:
(a) a VHH sequence as shown in SEQ. ID NO: 1, 5, 9, 13, 17, 21, 25, 29, or 33; and optionally an additional cysteine residue at its N- and/or C-terminus;
(b) a VHH sequence which has an identity of at least 70%, at least 80%, at least 90%, at least 95% or at least 99% to a VHH sequence of (a), or
(c) a VHH antibody, which competes with a VHH antibody of (a) for the binding to the respective component of the NPC.

6. The set of any one of claims 1-5 wherein at least one VHH antibody carries a labelling agent, wherein the labelling agent is particularly attached to a solvent-accessible ectopic SH group on the VHH antibody.

7. The set of claim 6 wherein at least 2 VHH antibodies carry different labelling agents, particularly different spectrally separatable fluorophores.

8. The set of any one of claims 1-7 wherein at least one VHH antibody is a thermostabilized VHH antibody which comprises two structural cysteines that are quantitatively oxidized and form an intramolecular disulfide bond.

9. Use of the set of any one of claims 1-8 for imaging applications particularly for fluorescence spectroscopy or microscopy.

10. The use of claim 9 wherein the set further comprises a secondary detection antibody.

11. The use of claim 10, wherein the secondary detection antibody is a VHH antibody comprising
(a) a VHH sequence as shown in SEQ. ID NO: 37; and optionally an additional cysteine residue at its N- and/or C-terminus;
(b) a VHH sequence which has an identity of at least 70%, at least 80%, at least 90%, at least 95% or at least 99% to a VHH sequence of (a), or
(c) a VHH antibody, which competes with a VHH antibody of (a) for the binding to its target antigen.

12. A method of producing a stable VHH antibody comprising the steps:
(i) expressing a VHH antibody comprising two structural cysteines and optionally at least one solvent-accessible ectopic cysteine in a host cell under reducing or at least partially reducing conditions,
(ii) purifying the VHH antibody from the host cell or the culture medium under conditions wherein the at least one solvent-acessible ectopic cysteine, if present, is in the reduced SH state;
(iii) optionally attaching a labelling agent to the at least one solvent-accessible ectopic cysteine under conditions while keeping the two structural cysteines unmodified,
(iv)subjecting the VHH antibody to an oxidation that converts the two structural cysteines to a disulfide bond, and
(v) obtaining a thermostabilized VHH antibody wherein the two structural cysteines are quantitatively oxidized to a disulfide bond

13. The method of claim 12 wherein step (iv) comprises a treatment of the VHH antibody with an oxidant which is a disulfide compound particularly oxidized glutathione (GSSG) optionally in the presence of a protein disulfide isomerase (PDI), particularly in the presence of DsbA and/or DsbC from *E. coli.*

14. The method of claim 12 or 13 wherein step (iv) comprises a transient thermal unfolding of the VHH antibody.

15. The method of claim 14 wherein the thermal unfolding comprises incubating the VHH antibody at a temperature which is in the range between the onset of melting of the reduced form of the VHH antibody and the nominal melting temperature (Tm) of the reduced form of the VHH antibody.
